# EUROPÄISCHE PATENTSCHRIFT

(11) **EP 3 586 110 B1**
(45) Veröffentlichungstag und Bekanntmachung des Hinweises auf die Patenterteilung: **18.06.2025**
(21) Anmeldenummer: 18704961.4
(22) Anmeldetag: 09.02.2018
(51) Int. Cl.: G01N 21/77, A61B 5/00

(54) **VERFAHREN UND VORRICHTUNG ZUM ERMITTELN EINES INDIVIDUELL FÜR EINEN NUTZER ABGELEITETEN PFLEGEPRODUKTS ANHAND EINES ERMITTELTEN HAUTFETTGEHALTS**
METHOD AND DEVICE FOR DETERMINING A CARE PRODUCT INDIVIDUALLY DERIVED FOR A USER ON THE BASIS OF A DETERMINED SKIN FAT CONTENT
PROCÉDÉ ET DISPOSITIF POUR DÉTERMINER UN PRODUIT DE SOIN DÉRIVÉ INDIVIDUELLEMENT POUR UN UTILISATEUR À PARTIR D'UNE TENEUR EN GRAISSE DE LA PEAU DÉTERMINÉE

(30) Priorität: 22.02.2017 DE 102017202856
(43) Veröffentlichungstag der Anmeldung: 01.01.2020
(73) Patentinhaber: Henkel AG & Co. KGaA, 40589 Düsseldorf (DE)
(72) Erfinder: BOCK, Andreas, 41464 Neuss (DE); WALDMANN-LAUE, Marianne, 40789 Monheim (DE); WELSS, Thomas, 40591 Düsseldorf (DE)
(86) Internationale Anmeldenummer: PCT/EP2018/053298
(87) Internationale Veröffentlichungsnummer: WO 2018/153693

(56) Entgegenhaltungen:
- WO-A1-93/14699
- KR-A- 20160 038 109
- KR-B1- 101 318 607
- US-A- 4 483 619
- US-A- 4 494 869
- US-A1- 2010 105 102
- MSABBRI A R ET AL: "Development of optical near-infrared spectroscopy instruments for human skin sebum measurement", PROCEEDINGS OF SPIE, IEEE, US, vol. 9129, 8 May 2014 (2014-05-08), pages 91293M - 91293M, XP060038883, ISBN: 978-1-62841-730-2, DOI: 10.1117/12.2052809
- COURAGE+KHAZAKA ELECTRONIC GMBH: "Sebum Measurement App for Smart-Phones", 7 January 2017 (2017-01-07), XP055472369, Retrieved from the Internet <URL:https://web.archive.org/web/20170107150122/http://www.courage-khazaka.de/index.php/en/news/news-for-the-point-of-sale-measurements/325-sebum-app-e> [retrieved on 20180503]

## Beschreibung

Die Erfindung betrifft ein Verfahren gemäß Anspruch 1 und eine Vorrichtung gemäß Anspruch 10 zum Ermitteln eines individuell für einen Nutzer abgeleiteten Pflegeprodukts Gesunde und jung aussehende Haut (normale Haut) kann ein kleinporiges, straffes Erscheinungsbild aufweisen. Die normale Haut kann sich geschmeidig anfühlen und weder zu trocken noch zu fett sein.

Im Gegensatz dazu kann trockene Haut im Erscheinungsbild schuppig, rau und/oder empfindlich gegenüber äußeren Einflüssen sein. Oftmals können ältere Menschen von trockener Haut betroffen sein.

Trockene Haut kann beispielsweise durch eine Störung einer Barrierefunktion der Haut wegen einer ungeeigneten Menge an Hautlipiden und/oder aufgrund einer falschen Zusammensetzung der Hautlipide zustande kommen.

Für eine Pflege speziell von trockener Haut können verschiedene spezielle Reinigungs- und Pflegeprodukte für das Gesicht und/oder den Körper erhältlich sein.

Bei fettiger Haut, die beispielsweise insbesondere in den Jahren nach der Pubertät auftreten kann, z.B. vor allem im Gesicht (da hier viele Talgdrüsen vorhanden sind), kann auf der Hautoberfläche ein Überschuss an Hautlipiden vorhanden sein. Dadurch kann ein Wachstum von Mikroorganismen gefördert werden, was zu unreiner Haut führen kann. Die fettige Haut kann grobporig und glänzend erscheinen.

Auch für eine Pflege speziell von fettiger Haut können verschiedene spezielle Reinigungs- und Pflegeprodukte, insbesondere für das Gesicht, erhältlich sein.

Bei einem als Mischhaut bezeichneten Hautzustand können sowohl trockene als auch fettige Hautbereiche vorhanden sein. Diese können sich beispielsweise abwechseln. Ein fettiger Bereich kann beispielsweise in einer so genannten T-Zone vorliegen, welche einen Stirnbereich, einen Nasenbereich und einen Kinnbereich aufweisen kann, während ein trockener Bereich beispielsweise die restlichen Bereiche im Gesicht aufweisen kann.

Auch für eine Pflege speziell von Mischhaut können verschiedene spezielle Reinigungs- und Pflegeprodukte, insbesondere für das Gesicht, erhältlich sein.

Ohne fachkundige dermatologische oder kosmetische Beratung kann es für einen Nutzer schwer sein, seinen individuellen Hautzustand und die für seinen Hautzustand geeigneten Kosmetika zu ermitteln.

Sofern der Nutzer dennoch kosmetische Produkte konsumiert, kann es praktisch unmöglich sein, einen Behandlungserfolg nachzuvollziehen, weil es dem Nutzer, z.B. zu Hause, an Möglichkeiten fehlt, standardisiert und objektiv ein Behandlungsergebnis zu beurteilen.

Dadurch kann es dem Konsumenten erschwert sein, eine individuelle Wirksamkeit eines Kosmetikums zu beurteilen, was dazu führen kann, dass eine Motivation, eine entsprechende kosmetische Behandlung, beispielsweise längerfristig, durchzuführen, beeinträchtigt sein kann. Dies kann selbst dann der Fall sein, wenn ein Kosmetikprodukt geeignet wäre, eine objektiv nachweisbare erwünschte Wirkung zu erzielen. In vielen Bereichen des täglichen Lebens gibt es seit einiger Zeit einen Trend zu personalisierten Programmen, die auf individuelle Voraussetzungen und Bedürfnisse gezielt eingehen können, beispielsweise in einem Ernährungs- oder Gesundheitsbereich, aber auch in einem Bereich personalisierter Kosmetik. Diese kann es einem Nutzer ermöglichen, gezielt Kosmetikprodukte zu finden und/oder Pflegehinweise zu erhalten, die auf individuelle Bedürfnisse seiner Haut abgestimmt sind, und somit eine besonders hohe Wirksamkeit ermöglichen.

Dokument US4494869 A beschreibt ein Verfahren zur Messung des Hautfettgehaltes mit Hilfe eines optischen Gerätes, das Licht auf einen Detektor fokussiert. Das Licht durchdringt eine Platte, die mit der Haut in Kontakt war. Dokument US4483619 A beschreibt ein Verfahren zur Messung des Hautfettgehaltes, bei der Lichtimpulse durch eine Platte geleitet werden, die mit der Haut in Kontakt war. Die Dokumente KR20160038109 A und KR 101318670 B1 beschreiben jeweils Verfahren zur Messung von Hauteigenschaften mit Hilfe von Elektroden. Dokument US 2010105102 A1 beschreibt ein zur Identifizierung biologischer Marker für die Analyse von Haut- und Haarzuständen mit Hilfe von Klebstoffschichten. Die Internetseite https://web.archive.org/web/20170107150122/http://www.courage-khazaka.de/index.php/en/news/news-for-the-point-of-sale-mesaurements/325-sebum-app-e beschreibt eine Anwendung, mit der der Hautfettgehalt mit Hilfe eines Smartphones bestimmt werden kann.

Erfindungsgemäß wird eine Vorrichtung gemäß Anspruch 10 zum Ermitteln eines Hautzustands eines Nutzers unter Einbeziehung eines ermittelten Hautfettgehalts bereitgestellt.

Die Vorrichtung kann in verschiedenen Ausführungsbeispielen günstig und klein (z.B. tragbar) ausgebildet sein. Die Vorrichtung kann an einem (lebenden) Nutzer angewendet werden, beispielsweise bei dem Nutzer zu Hause, in einem Verkaufspunkt für Kosmetika und/oder in einem Kosmetikstudio. Die Vorrichtung kann in verschiedenen Ausführungsbeispielen ein Smartphone, ein Tablet, ein iPad^{®} oder eine ähnliche elektronische Schaltkreisvorrichtung (z.B. eine Datenverarbeitungsvorrichtung und/oder eine Datenübertragungsvorrichtung) aufweisen, und eine Zusatzvorrichtung, die beispielsweise an der elektronischen Schaltkreisvorrichtung anbringbar und/oder damit verbindbar (z.B. mittels einer Datenverbindung verbindbar) sein kann. Die Zusatzvorrichtung (ggf. sogar die gesamte Vorrichtung) kann eine Größe aufweisen, die es ermöglicht, sie problemlos in einer Hand- oder Hosentasche unterzubringen, beispielsweise mit einer Fläche von weniger als 36 cm² und mit einer Dicke von weniger als 2 cm.

Die Vorrichtung weist eine Testfläche, eine Kamera und eine elektronische Schaltkreisvorrichtung und ist eingerichtet, mittels der Testfläche, der Kamera, der elektronischen Schaltkreisvorrichtung und einer Lichtquelle einen Hautfettgehalt (d.h. einen Gehalt von Hautlipiden auf/in der Haut) eines Nutzers zu ermitteln.

Die Testfläche ist derart gestaltet, dass sich ihre Lichtdurchlässigkeit in Abhängigkeit eines Fettgehalts von Haut, mit der sie in Kontakt gebracht wird, ändert, d.h. die Testfläche kann nach dem Kontakt mit (fetthaltiger) Haut lichtdurchlässiger oder weniger lichtdurchlässig werden, und zwar in stärkerem Maße, je fetthaltiger die Haut ist. Unter Verwendung des Hautfettgehalts wird ein Hautzustand des Nutzers ermittelt.

In verschiedenen Ausführungsbeispielen kann die Testfläche als ein sensorisches Material (z.B. eine Folie, eine Glas- oder Kunststoffplatte) gebildet sein, dessen Lichtdurchlässigkeit von einer Menge an Fett auf dem Material abhängen kann, was ein Messen der Hautfettmenge ermöglicht. Für das Messen kann das sensorische Material, ggf. mittels einer dafür vorgesehenen Halterung, mit der Haut des Nutzers in Kontakt gebracht werden (z.B. auf die Haut des Nutzers gedrückt werden), so dass das sensorische Material Hautfett aufnehmen kann. In verschiedenen Ausführungsbeispielen können dem Nutzer Instruktionen bereitgestellt werden hinsichtlich dessen, wie das In-Kontakt-Bringen auszuführen ist, z.B. hinsichtlich einer Kontaktdauer, eines Anpressdrucks, einer Relativbewegung (z.B. Drehen oder Wischen während des Drückens) bzw. eines Vermeidens einer Relativbewegung, usw.

In verschiedenen Ausführungsbeispielen kann die Vorrichtung eine Aufbewahrungsvorrichtung für die Testfläche aufweisen. Mittels der Aufbewahrungsvorrichtung kann die Testfläche beispielsweise vor Verschmutzung und/oder Verlust geschützt sein. In verschiedenen Ausführungsbeispielen (insbesondere, ohne darauf beschränkt zu sein, z.B. bei Verwendung von nur einmal verwendbaren Testflächen) kann die Aufbewahrungsvorrichtung als ein Raum zum Lagern der (z.B. einzeln) entnehmbaren und zur einmaligen Verwendung nutzbaren Testflächen gestaltet sein. In verschiedenen Ausführungsbeispielen (insbesondere, ohne darauf beschränkt zu sein, beispielsweise bei Nutzung einer mit der Halterung fest verbundenen, mehrfach verwendbaren Testfläche) kann die Aufbewahrungsvorrichtung so gestaltet sein, dass sie eine entfernbare Schutzvorrichtung aufweist, z.B. eine wegklappbare oder wegschwenkbare Schutzkappe oder ähnliches.

Erfindungsgemäß wird oder ist die Testfläche, d.h. das sensorische Material, nach dem In-Kontakt-Bringen mit der Haut derart angeordnet, dass die Kamera der Vorrichtung eingerichtet ist, von der Lichtquelle abgestrahltes Licht nach einem Hindurchtreten durch die Testfläche zu empfangen.

In verschiedenen Ausführungsbeispielen kann das sensorische Material (die Testfläche) von einem Rest der Vorrichtung trennbar sein. Dadurch kann ermöglicht sein, das sensorische Material auf die Haut zu drücken, während es von dem Rest der Vorrichtung getrennt ist, und danach das sensorische Material (die Testfläche) an oder in dem Rest der Vorrichtung anzuordnen, beispielsweise in einer Halterung (z.B. kann in einem Fall, in dem die Halterung eine Führung bereitstellt, das sensorische Material in diese eingeführt werden).

In verschiedenen Ausführungsbeispielen kann das sensorische Material (die Testfläche) auch während des In-Kontakt-Bringens mit der Haut mit dem Rest der Vorrichtung körperlich (z.B. fest) verbunden sein.

In verschiedenen Ausführungsbeispielen kann mittels der Vorrichtung zum Ermitteln eines Hautzustands der Hautfettgehalt des Nutzers photometrisch bestimmt werden. Für das photometrische Bestimmen des Hautfettgehalts des Nutzers kann in verschiedenen Ausführungsbeispielen die Testfläche in Richtung zur Kamera von einer Lichtquelle mit Licht bestrahlt werden.

In verschiedenen Ausführungsbeispielen kann eine Lichtmenge ermittelt werden, welche nach einem Hindurchtreten durch die nach dem In-Kontakt-Bringen mit der Haut ggf. mit Hautfett des Nutzers versehene Testfläche erreicht.

Die ermittelte Lichtmenge wird erfindingsgemäß mit einer Referenzlichtmenge verglichen. Die Referenzlichtmenge entspricht der Lichtmenge, welche die Kamera durch eine nicht kontaktierte, d.h. nicht mit Hautfett versehene, Testfläche erreichen würde.

Die Referenzlichtmenge kann in verschiedenen Ausführungsbeispielen, beispielsweise bei einer integrierten Lichtquelle oder einer anderen Lichtquelle, welche die Testfläche reproduzierbar immer auf im Wesentlichen gleiche Art beleuchtet (Lichtintensität, -wellenlänge, Beleuchtungsrichtung, Abstand zwischen Lichtquelle und Testfläche usw.), vorab, beispielsweise in einem Labor, ermittelt und bereitgestellt werden.

In verschiedenen Ausführungsbeispielen, z.B. bei einer Nutzung einer externen Lichtquelle, z.B. einer Lampe oder sonstigen allgemein verfügbaren Lichtquelle, kann die Referenzlichtmenge vor oder nach dem photometrischen Messen der mit Hautfett versehenen Testfläche ermittelt werden, z.B. unmittelbar davor oder (ggf. nach einem Reinigen der Testfläche, oder unter Verwendung einer hinsichtlich ihrer photometrischen Eigenschaften gleichartigen, nicht mit Hautfett versehenen Testfläche) danach.

Erfindungsgemäß wird unter Verwendung der Testfläche, welche mit der Haut des Nutzers in Kontakt war, ermittelte Lichtmenge mit der Referenzlichtmenge verglichen und anhand dessen ein Hautfettgehalt des Nutzers ermittelt, beispielsweise mittels eines Vergleichens mit Referenzdaten. Die Referenzdaten können in verschiedenen Ausführungsbeispielen vorab ermittelt worden sein, beispielsweise bei Laborversuchen.

In verschiedenen Ausführungsbeispielen kann das Vergleichen der ermittelten Lichtmenge mit der Referenzlichtmenge und/oder das Ermitteln der Hautfettmenge daraus mittels der elektronischen Schaltkreisvorrichtung (z.B. direkt) erfolgen, beispielsweise mittels einer App oder eines sonstigen Softwareprogramms, welches von der elektronischen Schaltkreisvorrichtung (z.B. einem Smartphone, einem Tablet, einem iPad^{®} oder ähnlichem) betrieben wird.

In verschiedenen Ausführungsbeispielen kann das Vergleichen der ermittelten Lichtmenge mit der Referenzlichtmenge und/oder das Ermitteln der Hautfettmenge daraus indirekt mittels der elektronischen Schaltkreisvorrichtung erfolgen, beispielsweise indem die elektronische Schaltkreisvorrichtung eine (z.B. kontaktlose) Datenkommunikationsverbindung bereitstellt und mittels der

Datenkommunikationsverbindung die Aufnahme der Lichtmenge (d.h. das mittels der Kamera aufgenommene digitale Bild), die ermittelte Lichtmenge und/oder das Ergebnis des Vergleichs der Lichtmenge mit der Referenzlichtmenge einer externen Datenverarbeitungsvorrichtung (z.B. einer Cloud) bereitstellt und von der externen Datenverarbeitungsvorrichtung den mittels der Aufnahme der Lichtmenge, der ermittelten Lichtmenge und/oder des Ergebnisses des Vergleichs der Lichtmenge mit der Referenzlichtmenge ermittelten Hautfettgehalt empfängt.

Dem Nutzer kann der ermittelte Hautfettgehalt in verschiedenen Ausführungsbeispielen auf beliebige, z.B. vom Nutzer wählbare, Art bereitgestellt werden, z.B. als ein Zahlenwert (eine Hautfettigkeitsmaßzahl), eine graphische Darstellung (beispielsweise einer Darstellung des ermittelten Werts in im Verhältnis zu einem gesamten Wertebereich von sehr trocken über normal bis hin zu sehr fettig), eine akustische Mitteilung, oder ähnliches.

Erfindungsgemäß ist die Vorrichtung ferner eingerichtet, basierend auf dem ermittelten Hautfettgehalt einen Hautzustand zu ermitteln. In einem Fall, in dem für nur einen Hautbereich der Hautfettgehalt ermittelt wird bzw. wurde, kann der Hautzustand, der basierend darauf ermittelt wird, als einheitlicher Hautzustand für den einen ermittelten Hautfettgehalt ermittelt werden, z.B. sehr trockene Haut bei sehr geringem Hautfettgehalt, sehr fettige Haut bei sehr hohem Hautfettgehalt, normale Haut bei normalem Hautfettgehalt, usw., mit einer beliebigen Anzahl von Abstufungen (z.B. dazwischen). In einem Fall, in dem für eine Mehrzahl von Hautbereichen der Hautfettgehalt ermittelt wird bzw. wurde, kann der Hautzustand, der basierend darauf ermittelt wird, als einheitlicher Hautzustand für die Mehrzahl von ermittelten Hautfettgehalten ermittelt werden, z.B. als extreme Mischhaut mit Bereichen sehr trockener Haut und Bereichen sehr fettiger Haut (z.B. bei für mindestens einen ersten Hautbereich ermitteltem sehr geringem Hautfettgehalt und für mindestens einen zweiten Hautbereich ermitteltem sehr hohem Hautfettgehalt), als leichte Mischhaut mit Bereichen trockener Haut und Bereichen fettiger Haut (z.B. bei für mindestens einen ersten Hautbereich ermitteltem geringem Hautfettgehalt und für mindestens einen zweiten Hautbereich ermitteltem hohem Hautfettgehalt), Mischhaut mit trockener Tendenz (z.B. bei für mindestens einen ersten Hautbereich ermitteltem sehr geringem Hautfettgehalt und für mindestens einen zweiten Hautbereich ermitteltem hohem Hautfettgehalt), Mischhaut mit fettiger Tendenz (z.B. bei für mindestens einen ersten Hautbereich ermitteltem geringem Hautfettgehalt und für mindestens einen zweiten Hautbereich ermitteltem sehr hohem Hautfettgehalt), usw.

In verschiedenen Ausführungsbeispielen kann der Hautzustand numerisch angegeben werden, beispielsweise als ein Summenwert, wodurch eine Plakativität (z.B. der Darstellung des Ergebnisses) erhöht werden kann.

Insbesondere in einem Fall, in welchem der Hautfettgehalt für eine Mehrzahl von Hautbereichen ermittelt wird, kann das Bereitstellen des Hautzustands an den Nutzer eine graphische Darstellung aufweisen, beispielsweise eine Anzeige, z.B. mittels einer Anzeigevorrichtung. In der graphischen Darstellung kann der ermittelte Hautfettgehalt der Mehrzahl von Bereichen mit einer Codierung anhand des Hautfettgehalts in einer Darstellung des Nutzers (z.B. einer schematischen Darstellung oder auf einem Foto des Nutzers) angezeigt werden. Beispielsweise können in einer schematischen Darstellung eines Gesichts des Nutzers Bereiche unterschiedlichen Hautfettgehalts mit unterschiedlichen Farben und/oder Mustern dargestellt werden, z.B. Bereiche mit normalem Hautfettgehalt grün, mit niedrigem Hautfettgehalt rot und mit hohem Hautfettgehalt blau oder ähnliches. In einem anderen Beispiel können einem Foto des Nutzers, z.B. einem digitalen Foto, welches das Gesicht des Nutzers zeigt, unterschiedliche Muster überlagert sein, z.B. Tropfenmuster für Bereiche mit hohem Hautfettgehalt und ein Linienmuster, welches eine aufreißende Fläche darstellt, für Bereiche mit niedrigem Hautfettgehalt, wobei Bereiche normaler Haut ungemustert verbleiben können, oder ähnliches.

Beim graphischen Darstellen des Hautzustands, z.B. des Hautfettgehalts des mindestens einen Hautbereichs, z.B. der Mehrzahl der Hautbereiche, kann in verschiedenen Ausführungsbeispielen nur für den Bereich/die Bereiche, für den/die der Hautfettgehalt ermittelt wurde, der Hautfettgehalt dargestellt werden. In verschiedenen Ausführungsbeispielen kann ein Bereich, für den ein ermittelter Hautfettgehalt angezeigt wird, über den Hautbereich hinaus, für den der Hautfettgehalt ermittelt wurde, extrapoliert werden, beispielsweise unter Einbeziehung typischer Hautfettverteilungsmuster. Beispielsweise kann unter Verwendung eines ermittelten Hautfettwerts für einen ersten Hautbereich innerhalb der T-Zone und eines zweiten Hautfettwerts für einen zweiten Hautbereich außerhalb der T-Zone der gesamten T-Zone der Hautfettwert des ersten Bereichs zugeordnet und in der graphischen Darstellung entsprechend dargestellt werden, und dem gesamten (Gesichts-)Hautbereich außerhalb der T-Zone kann der Hautfettwert des zweiten Bereichs zugeordnet und in der graphischen Darstellung entsprechend dargestellt werden.

Dabei kann in verschiedenen Ausführungsbeispielen eine Information darüber, wo der Bereich/die Bereiche ist/sind, der elektronischen Schaltkreisvorrichtung bereitgestellt werden, beispielsweise mittels des Nutzers, z.B. mittels einer Eingabevorrichtung (z.B. Antippen eines berührungsempfindlichen Displays, auf welchem eine schematische oder fotografische Darstellung des Nutzers angezeigt wird, mittels Texteingabe über eine Tastatur oder jede andere geeignete Eingabemöglichkeit).

In verschiedenen Ausführungsbeispielen kann bei Vorliegen einer Mehrzahl von Hautfettwerten für eine Mehrzahl von Hautbereichen das Darstellen der Verteilung der Hautfettwerte unter einer Annahme erfolgen, dass der Bereich, für den ein höherer Hautfettwert ermittelt wird, sich in der T-Zone befindet, und der Bereich, für den ein niedrigerer Hautfettwert ermittelt wird, sich außerhalb der T-Zone (aber z.B. innerhalb des Gesichts) befindet, und dementsprechend kann die (z.B. extrapolierte) Darstellung erfolgen.

In verschiedenen Ausführungsbeispielen kann eine Begründetheit der Annahme dadurch erhöht werden, dass der Nutzer vor dem Ermitteln seines Hautzustands instruiert wird (z.B. mittels der App bzw. Software), als den Hautbereich bzw. die Hautbereiche bestimmte Hautbereiche auszuwählen, z.B. den ersten Hautbereich innerhalb der T-Zone (z.B. an einer bestimmten Stelle innerhalb der T-Zone, z.B. an der Stirn oder am Kinn) und den zweiten Hautbereich außerhalb der T-Zone, z.B. an der Wange.

In verschiedenen Ausführungsbeispielen kann die Anzeigevorrichtung Teil der Vorrichtung sein, z.B. ein Display eines Smartphones, Tablets oder iPads^{®}. In verschiedenen Ausführungsbeispielen kann die Anzeigevorrichtung mit der Vorrichtung gekoppelt sein, beispielsweise mittels einer (z.B. kabellosen) Datenverbindung.

Zusätzlich oder alternativ kann in verschiedenen Ausführungsbeispielen dem ermittelten Hautzustand ein Qualitätswert zugeordnet werden, mit einem hohen Qualitätswert für normale/gesunde Haut und einem niedrigen Qualitätswert für zu trockene/zu fettige Haut.

In verschiedenen Ausführungsbeispielen kann die elektronische Schaltkreisvorrichtung eingerichtet sein, selbst, d.h. direkt, den mindestens einen Hautzustand zu ermitteln, beispielsweise mittels einer Software, z.B. einer App, die auf der elektronischen Schaltkreisvorrichtung installiert sein kann.

In verschiedenen Ausführungsbeispielen kann die elektronische Schaltkreisvorrichtung eingerichtet sein, indirekt den mindestens einen Hautzustand zu ermitteln, beispielsweise indem die elektronische Schaltkreisvorrichtung das Ermitteln des Hautzustands mittels der externen Datenverarbeitungsvorrichtung, z.B. mittels einer Software, z.B. einer App, die auf der externen Datenverarbeitungsvorrichtung installiert sein kann, veranlasst, beispielsweise ähnlich wie oben für das Ermitteln des Hautfettgehalts beschrieben.

In verschiedenen Ausführungsbeispielen kann die Software/App zum Ermitteln des Hautfettgehalts dieselbe sein wie die Software/App zum Ermitteln des Hautzustands. In verschiedenen Ausführungsbeispielen, z.B. in einem Fall, dass der Hautfettgehalt mittels der externen Datenverarbeitungsvorrichtung ermittelt wird, wohingegen der Hautzustand von der elektronische Schaltkreisvorrichtung ermittelt wird, oder umgekehrt, kann/können unterschiedliche Software/Apps für das Ermitteln des Hautfettgehalts und für das Ermitteln des Hautzustands verwendet werden.

Erfindungsgemäß werden ein Verfahren gemäß Anspruch 1 und eine Vorrichtung gemäß Anspruch 10 zum Ermitteln eines Hautzustands bereitgestellt. Dafür wird ein Hautfettgehalt ermittelt. Die Vorrichtung, das Verfahren, und/oder der mittels der Vorrichtung bzw. des Verfahrens ermittelte Hautzustand kann/können genutzt werden, um die Bedürfnisse der Haut des Nutzers zu ermitteln.

In verschiedenen Ausführungsbeispielen soll es einem Nutzer ermöglicht werden, gezielt kosmetische Produkte zu finden und/oder Pflegeratschläge zu erhalten, die auf individuelle Bedürfnisse einer Haut des Nutzers abgestimmt sein können.

Für das Ermitteln des Hautfettgehalts und/oder für das Ermitteln des Hautzustands kann in verschiedenen Ausführungsbeispielen eine Software, beispielsweise eine App, genutzt werden, welche beispielsweise auf einer tragbaren Datenverarbeitungsvorrichtung (z.B. einem Smartphone, einem Tablet, einem iPad^{®} oder ähnlichem) installiert sein kann. Eine Kamera, z.B. für den Bereich sichtbaren Lichts, kann an die Datenverarbeitungsvorrichtung angeschlossen oder darin integriert sein. Mittels der Kamera kann der Lipidgehalt der Haut ermittelt werden. Ferner kann, beispielsweise anhand einer Hautfettgehaltmessung für einen einzelnen Hautbereich oder anhand einer Mehrzahl von Hautfettgehaltmessungen für unterschiedliche Hautbereiche, ein Hautzustand ermittelt werden, z.B. fettige Haut, normale Haut, trockene Haut oder Mischhaut, welche sowohl Bereiche trockener Haut als auch Bereiche fettiger Haut aufweist.

In verschiedenen Ausführungsbeispielen können mittels der tragbaren Datenverarbeitungsvorrichtung, z.B. mittels der Software/App, der ermittelte Hautfettgehalt und/oder der ermittelte Hautzustand als Werte (z.B. mit willkürlichen Einheiten), als verbale Mitteilung, als graphische Darstellung (z.B. als schematische bildliche Darstellung, welche Hautbereiche fettige/trockene/normale Haut aufweisen), o.ä. dargestellt bzw. mitgeteilt werden.

In verschiedenen Ausführungsbeispielen kann dem Nutzer, basierend auf dem ermittelten Hautzustand, mindestens ein Kosmetikprodukt empfohlen werden. Das Kosmetikprodukt kann geeignet sein, den Hautzustand des Nutzers zu erhalten oder zu verbessern (z.B. einen Hautfettgehalt zu normalisieren). In verschiedenen Ausführungsbeispielen kann das Kosmetikprodukt eine Creme, eine Lotion, eine Salbe, ein Öl, eine Seife oder ähnliches aufweisen.

In verschiedenen Ausführungsbeispielen können anhand des ermittelten Hautfettgehalts und/oder anhand des ermittelten Hautzustands Produktempfehlungen für individuell für den Nutzer passende Pflegeprodukte und/oder individuelle Pflegehinweise abgeleitet werden. Die Produktempfehlungen und/oder Pflegehinweise können beispielsweise mittels einer Software, z.B. einer App, bereitgestellt werden.

Immer mehr Kunden wünschen sich ein individuell auf ihre Bedürfnisse abgestimmtes Produkt. Dabei kann es sich um ein speziell für den Kunden hergestelltes Produkt oder ein sogenanntes "mass customized" Produkt handeln. Bei einem "mass customized" Produkt kann durch Variation von wenigen, aus Kundensicht jedoch entscheidenden Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurator abläuft. Dieser Konfigurator hilft dem Kunden bei der Auswahl der Merkmale/Inhaltsstoffe und weist ihn auf die zulässigen/unzulässigen Merkmalskombinationen hin, wobei letztere dann nicht ausgewählt werden können.

Pflegeprodukte für die Haut umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten Hautfettgehalt und/oder Hautzustand ungeeigneter Inhaltsstoffe vermieden werden. Umgekehrt kann die Auswahl für den ermittelten Hautfettgehalt und/oder Hautzustand geeigneter Inhaltsstoffe durch den Produktkonfigurator vorgegeben oder vorgeschlagen werden.

In verschiedenen Ausführungsbeispielen kann die Software/App, welche den Hautfettgehalt und/oder den Hautzustand ermittelt, dieselbe sein, die die Produktempfehlung und/oder die Pflegehinweise ermittelt. In verschiedenen Ausführungsbeispielen können unterschiedliche Softwareprogramme/Apps verwendet werden für einen Teil der verschiedenen Vorgänge oder alle verschiedenen Vorgänge (Ermitteln des Hautfettgehalts, Ermitteln des Hautzustands, Ermitteln einer Produktempfehlung, Ermitteln eines Pflegehinweises).

In verschiedenen Ausführungsbeispielen kann ein Behandlungserfolg bei einer kosmetischen Behandlung, welche ein positives Beeinflussen des ermittelten Hautfettgehalts bzw. des ermittelten Hautzustands zum Ziel haben kann, überwacht werden. In verschiedenen Ausführungsbeispielen kann die Software/App eine Kontrolle und/oder Nachverfolgung der Ergebnisse mittels einer Darstellung (z.B. einer graphischen Darstellung) der Messergebnisse im Verlauf der Zeit ermöglichen.

In verschiedenen Ausführungsbeispielen können beim Ermitteln der Produkt- und Pflegeempfehlungen ferner Informationen hinsichtlich Alter, Geschlecht, eines generellen Gesundheitszustands, eines Hautzustands, Ernährungsgewohnheiten und weiterer Verhaltensweisen des Nutzers (z.B. tägliche Aufenthaltsdauer im Freien/in der Sonne/im Wasser, Rauchgewohnheiten usw.) verwendet werden, z.B. mittels der Software/App. Diese Informationen können in verschiedenen Ausführungsbeispielen mittels der Software/App vom Nutzer erfragt werden.

In verschiedenen Ausführungsbeispielen können zum Beurteilen einer Eignung eines Pflegeprodukts und/oder eines Pflegehinweises zum Pflegen einer Haut mit einem gegebenen Hautzustand Literaturdaten zu Grunde gelegt werden.

In verschiedenen Ausführungsbeispielen kann jedem Hautzustand einer Mehrzahl von Hautzuständen ein Qualitätswert zugeordnet sein oder werden. Ein Pflegeprodukt und/oder ein Pflegehinweis kann für einen Hautzustand als geeignet bewertet werden, wenn zu erwarten ist, z.B. aufgrund von Literaturdaten, Versuchsergebnissen oder Erfahrungswerten (welche beispielsweise fortlaufend, z.B. auch während einer Nutzung der Software/der App durch den Nutzer, mit neuen Erfahrungswerten anderer Nutzer aktualisiert werden können, siehe unten), dass bei einer (z.B. regelmäßigen) Anwendung des Pflegeprodukts und/oder des Pflegehinweises der Hautzustand des Nutzers aufrechterhalten wird oder sich zu einem Hautzustand mit einem höheren (d.h. besseren) Qualitätswert ändert.

In verschiedenen Ausführungsbeispielen kann eine Beurteilung einer Eignung eines Pflegeproduktes, einen Hautzustand zu verbessern, bestätigt oder abgeändert werden mittels eines Aufnehmens von Erfahrungswerten weiterer Nutzer mit demselben oder einem ähnlichen Hautzustand, beispielsweise von Erfahrungswerten hinsichtlich eines Behandlungserfolgs. Damit kann ermöglicht sein, dass der Nutzer immer eine optimale Empfehlung erhält.

In verschiedenen Ausführungsbeispielen können eine Kontrolle und eine Nachverfolgung einer Wirksamkeit einer kosmetischen Behandlung auf objektive und standardisierte Art und Weise ermöglicht sein. Die kosmetische Behandlung kann in verschiedenen Ausführungsbeispielen das Ziel haben, einen Hautfettgehalt zu normalisieren.

In verschiedenen Ausführungsbeispielen kann eine Wirksamkeit einer (z.B. kosmetischen) Behandlung besser nachvollzogen werden und dadurch eine Auswahl eines individuell geeigneten Produkts vereinfacht sein oder werden.

In verschiedenen Ausführungsbeispielen kann eine Motivation eines Nutzers erhöht werden, eine kosmetische Behandlung längerfristig durchzuführen, beispielsweise mittels eines Vergleichs mit anderen Nutzern, z.B. mittels von den anderen Nutzern bereitgestellten Informationen über Behandlungserfolge.

In verschiedenen Ausführungsbeispielen kann die Pflegeempfehlung den Besuch eines Hautarzt oder eines Kosmetikers umfassen. In verschiedenen Ausführungsbeispielen kann direkt über die Software/App, die Produktempfehlung und/oder die Pflegehinweise ermittelt, ein Buchungsvorgang eingeleitet werden. Dazu können beispielsweise in der Software/App die Kontaktdaten von Hautärzten und/oder Kosmetikern hinterlegt sein und diese dem Nutzer angezeigt werden. Zusätzlich kann über Filter, wie beispielsweise die Postleitzahl, die Auswahl eingeschränkt werden. In verschiedenen Ausführungsbeispielen kann direkt über die Software/App eine Terminbuchung vorgenommen werden. Alternativ kann die Buchung eines Termins über eine separate Software/App, wie beispielsweise Treatwell, erfolgen.

Erfindungsgemäß wird wird ein Verfahren zum Ermitteln eines Hautzustands gemäß Anspruch 1 bereitgestellt, umfassend: ein Ermitteln eines Hautfettgehalts, aufweisend: für mindestens einen Hautbereich eines Nutzers, Kontaktieren des Hautbereichs mittels einer Testfläche, wobei die Testfläche derart gestaltet ist, dass ihre Lichtdurchlässigkeit sich in Abhängigkeit eines Hautfettgehalts ändert, Aufnehmen einer Menge von Licht mittels einer digitalen Kamera, wobei das aufgenommene Licht von einer Lichtquelle abgestrahlt wird, durch die Testfläche hindurchtritt und die Kamera erreicht, wobei die digitale Kamera Teil einer tragbaren Vorrichtung ist, Vergleichen der Menge des aufgenommenen Lichts mit einer Referenzlichtmenge für eine unkontaktierte Testfläche, und Ermitteln des Hautfettgehalts unter Verwendung des Vergleichs, und Ermitteln des Hautzustands basierend auf dem ermittelten Hautfettgehalt, und Ermitteln des Pflegeprodukts mittels eines Produktkonfigurators, wobei der Produktkonfigurator konfiguriert ist, eine Auswahl für den ermittelten Hautfettgehalt und/oder Hautzustand geeigneter Inhaltsstoffe auszugeben und die Auswahl chemisch und/ oder physikalisch inkompatibler Inhaltsstoffe oder die Auswahl für den ermittelten Hautfettgehalt und/oder Hautzustand ungeeigneter Inhaltsstoffe zu vermeiden

In verschiedenen Ausführungsbeispielen kann das Ändern der Lichtdurchlässigkeit ein Erhöhen der Lichtdurchlässigkeit mit Erhöhung des Hautfettgehalts aufweisen.

In verschiedenen Ausführungsbeispielen kann das Ändern der Lichtdurchlässigkeit ein Verringern der Lichtdurchlässigkeit mit Erhöhung des Hautfettgehalts aufweisen.

In verschiedenen Ausführungsbeispielen kann der mindestens eine Hautbereich eine Mehrzahl von Hautbereichen des Nutzers aufweisen.

In verschiedenen Ausführungsbeispielen kann die Mehrzahl von Hautbereichen mindestens einen ersten Gesichtshautbereich in einer Stirn, Nase und Kinn aufweisenden T-Zone und mindestens einen zweiten Gesichtshautbereich außerhalb der T-Zone aufweisen.

In verschiedenen Ausführungsbeispielen kann das Ermitteln des Hautfettgehalts für die Mehrzahl von Hautbereichen aufweisen: ein Kontaktieren eines ersten Hautbereichs mittels der Testfläche, ein Aufnehmen einer ersten Menge von Licht mittels der digitalen Kamera, wobei das aufgenommene Licht von der Lichtquelle abgestrahlt wird, durch die Testfläche hindurchtritt und die Kamera erreicht, ein Reinigen der Testfläche, ein Kontaktieren eines zweiten Hautbereichs mittels der gereinigten Testfläche, ein Aufnehmen einer zweiten Menge von Licht mittels der digitalen Kamera, wobei das aufgenommene Licht von der Lichtquelle abgestrahlt wird, durch die Testfläche hindurchtritt und die Kamera erreicht.

In verschiedenen Ausführungsbeispielen kann das Verfahren ferner, vor dem Kontaktieren des Hautbereichs mittels der Testfläche, ein Aufnehmen der Referenzlichtmenge mittels der digitalen Kamera aufweisen, wobei das aufgenommene Licht von der Lichtquelle abgestrahlt wird, durch die unkontaktierte Testfläche hindurchtritt und die Kamera erreicht.

In verschiedenen Ausführungsbeispielen kann die tragbare Vorrichtung ein Smartphone, ein Tablet oder ein iPad^{®} aufweisen.

In verschiedenen Ausführungsbeispielen kann das Aufnehmen der Menge von Licht, das Vergleichen der Menge des aufgenommenen Lichts mit der Referenzlichtmenge, das Ermitteln des Hautfettgehalts und/ oder das Ermitteln des Hautzustands basierend auf dem ermittelten Hautfettgehalt mittels einer App erfolgen.

In verschiedenen Ausführungsbeispielen kann die Lichtquelle Teil der tragbaren Vorrichtung sein.

Die vorliegende Offenbarung bezieht sich auch auf ein Verfahren zum Ermitteln einer kosmetischen Hautbehandlungsempfehlung. Das Verfahren kann aufweisen: ein Ermitteln eines Hautzustands gemäß verschiedenen Ausführungsbeispeilen, und ein Ermitteln des Kosmetikprodukts und/oder einer Pflegeanweisung basierend auf dem ermittelten Hautzustand und einer Datenbank, welche eine Mehrzahl von Hautzuständen und eine Mehrzahl von zugeordneten Kosmetikprodukten und/oder Pflegeanweisungen aufweist, wobei jedem Hautzustand der Mehrzahl von Hautzuständen mindestens ein geeignetes Kosmetikprodukt und/oder mindestens eine Pflegeanweisung zugeordnet ist.

Jedem Hautzustand kann ein Qualitätswert zugeordnet sein, und das Kosmetikprodukt und/oder die Pflegeanweisung kann geeignet sein für mindestens einen Hautzustand der Mehrzahl von Hautzuständen, wenn basierend auf gespeicherten Erfahrungswerten mit dem Kosmetikprodukt eine Verbesserung oder Aufrechterhaltung des Qualitätswerts des Hautzustands zu erwarten ist.

Das Verfahren kann ferner ein Aktualisieren der Datenbank basierend auf neuen Erfahrungswerten von einer Mehrzahl von Nutzern aufweisen.

Das Ermitteln des Hautfettgehalts und/oder das Ermitteln des Hautzustands ein Übertragen der Aufnahme, kann der ermittelten Menge des aufgenommenen Lichts, eines Ergebnisses des Vergleichs der Menge des aufgenommenen Lichts mit der Referenzlichtmenge und/oder des ermittelten Hautfettgehalts an eine externe Datenverarbeitungsvorrichtung und ein Empfangen des Hautfettgehalts und/oder des Hautzustands aufweisen.

In verschiedenen Ausführungsbeispielen können die elektronische Schaltkreisvorrichtung und die Kamera eine integrierte Einheit bilden.

In verschiedenen Ausführungsbeispielen kann die tragbare Vorrichtung ein Smartphone, ein Tablet oder ein iPad^{®} aufweisen.

In verschiedenen Ausführungsbeispielen kann die tragbare Vorrichtung ferner eine Datenaustauschvorrichtung zum kontaktlosen Datenaustausch aufweisen.

In verschiedenen Ausführungsbeispielen kann die Testfläche reinigbar und nach einem Reinigen als Testfläche wiederverwendbar sein.

In verschiedenen Ausführungsbeispielen kann die Testfläche durch eine aufgeraute Oberfläche gebildet sein.

In verschiedenen Ausführungsbeispielen kann die Vorrichtung ferner die Lichtquelle aufweisen, wobei die Kamera, die elektronische Schaltkreisvorrichtung und die Lichtquelle eine integrierte Einheit bilden.

In verschiedenen Ausführungsbeispielen kann die Vorrichtung ferner ein optisches Element aufweisen, wobei die Lichtquelle, das optische Element, die Testfläche und die Kamera derart anordenbar sind, dass für das Licht der Lichtquelle mittels des optischen Elements die Kamera nach dem Hindurchtreten durch die Testfläche erreichbar ist.

In den Zeichnungen beziehen sich ähnliche Bezugszeichen üblicherweise auf dieselben Teile in allen unterschiedlichen Ansichten, wobei der Übersichtlichkeit wegen teilweise darauf verzichtet wird, sämtliche einander entsprechenden Teile in allen Figuren mit Bezugszeichen zu versehen. Teile derselben oder ähnlicher Art können zur Unterscheidung zusätzlich zu einem gemeinsamen Bezugszeichen mit einer nachgestellten Ziffer oder einem nachgestellten kleinen Buchstaben versehen sein. Die Zeichnungen sollen nicht notwendigerweise eine maßstabgetreue Wiedergabe darstellen, sondern die Betonung liegt vielmehr auf einem Veranschaulichen der Prinzipien der Erfindung. In der folgenden Beschreibung werden verschiedene Ausführungsformen der Erfindung mit Bezug auf die folgenden Zeichnungen beschrieben, in denen:
FIG. 1A und FIG. 1B jeweils eine Vorrichtung zum Ermitteln eines Hautzustands zeigen und ein Verwenden der Vorrichtung veranschaulichen;
FIG. 2 Hautregionen zum Anwenden eines Verfahrens und einer Vorrichtung zum Ermitteln eines Hautzustands veranschaulicht:
FIG. 3A und FIG. 3B jeweils eine Vorrichtung zum Ermitteln eines Hautzustands zeigen;
FIG. 4A und FIG. 4B jeweils eine Testvorrichtung mit einer Testfläche zum Verwenden als Teil einer Vorrichtung zum Ermitteln eines Hautzustands zeigen;
FIG. 5 eine Veranschaulichung einer graphischen Darstellung eines Ergebnisses eines Verfahrens zum Ermitteln eines Hautzustands zeigt;
FIG. 6 ein Flussdiagramm zum Veranschaulichen eines Verfahrens zum Ermitteln eines Kosmetikprodukts zur Hautbehandlung zeigt;
FIG. 7 ein Flussdiagramm eines Verfahrens zum Ermitteln eines Hautzustands zeigt;
FIG. 8 ein Flussdiagramm eines Verfahrens zum Ermitteln einer kosmetischen Hautbehandlungsempfehlung zeigt; und

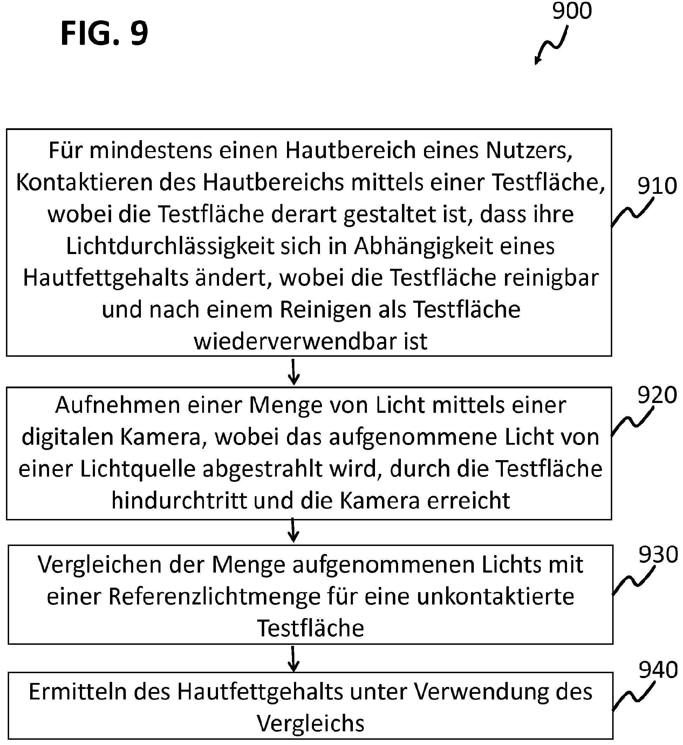

FIG. 9 ein Flussdiagramm eines Verfahrens zum Ermitteln eines Hautfettgehalts zeigt.

Die folgende ausführliche Beschreibung bezieht sich auf die begleitenden Zeichnungen, die als Beispiel durch Veranschaulichung bestimmte Details und Ausführungen zeigen, in denen die Erfindung in die Praxis umgesetzt werden kann.

Die Erfindung ist durch die angehängten Ansprüche definiert. Die folgende Beschreibung der Figuren dient lediglich dem besseren Verständnis der Erfindung. Das Wort "beispielhaft" wird hierin in der Bedeutung von "als ein Beispiel, ein Exemplar oder eine Veranschaulichung dienend" verwendet. Alle hierin als "beispielhaft" beschriebenen Ausführungsformen oder Ausgestaltungen sind nicht notwendigerweise als bevorzugt oder vorteilhaft anderen Ausführungsformen oder Ausgestaltungen gegenüber zu deuten.

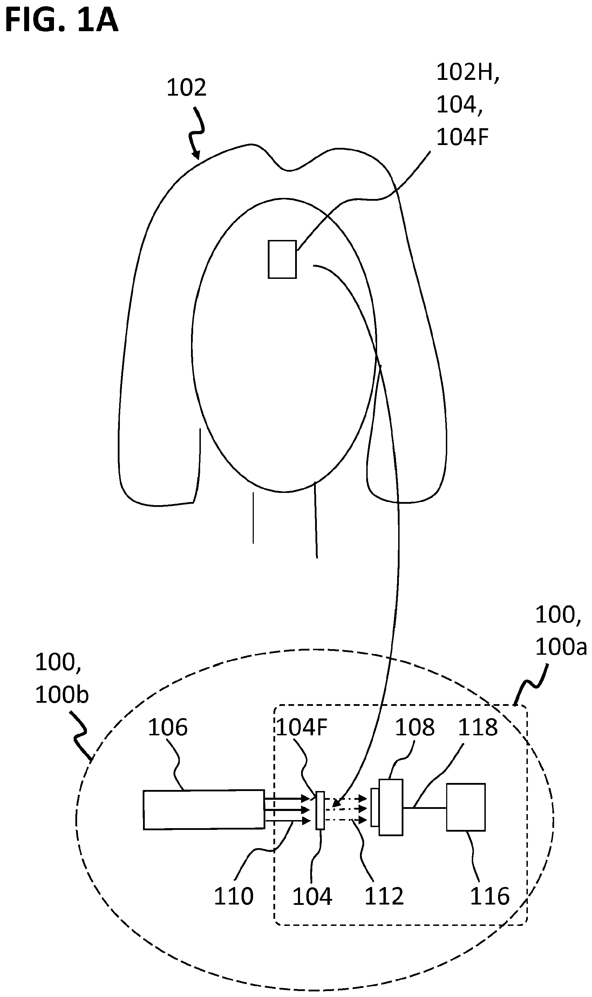

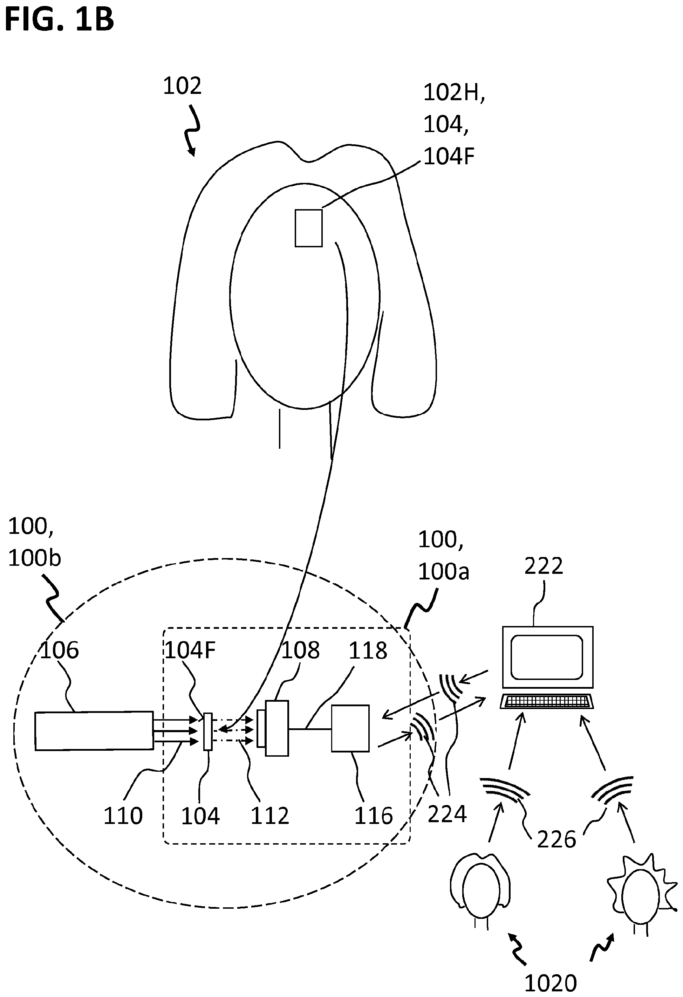

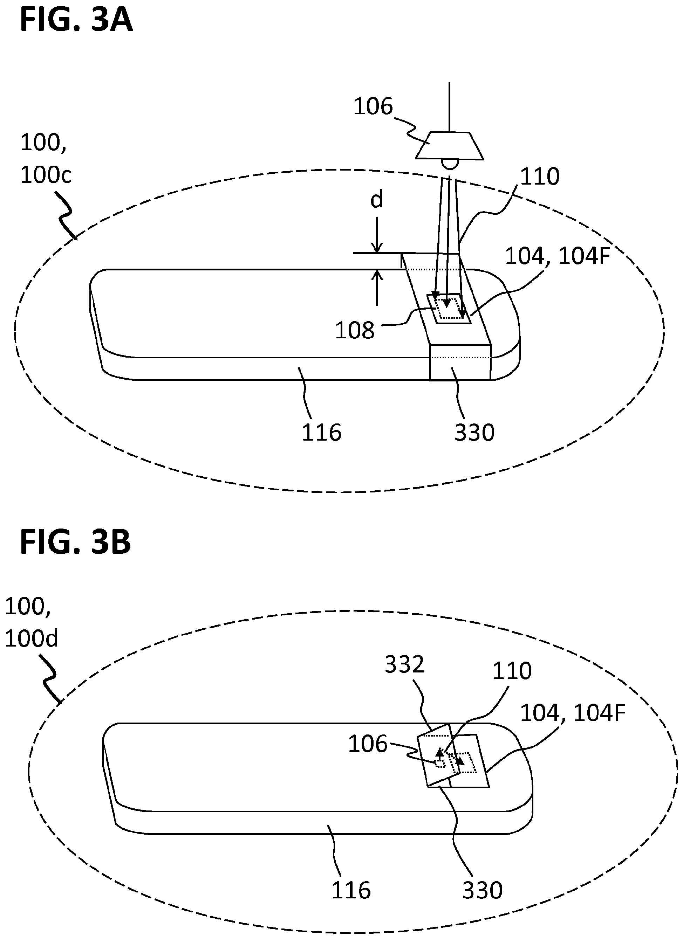

FIG. 1A, FIG. 1B, FIG. 3A und FIG. 3B zeigen jeweils eine Vorrichtung 100 (100a, 100b, 100c bzw. 100d) zum Ermitteln eines Hautzustands eines Nutzers 102 gemäß verschiedenen Ausführungsbeispielen.

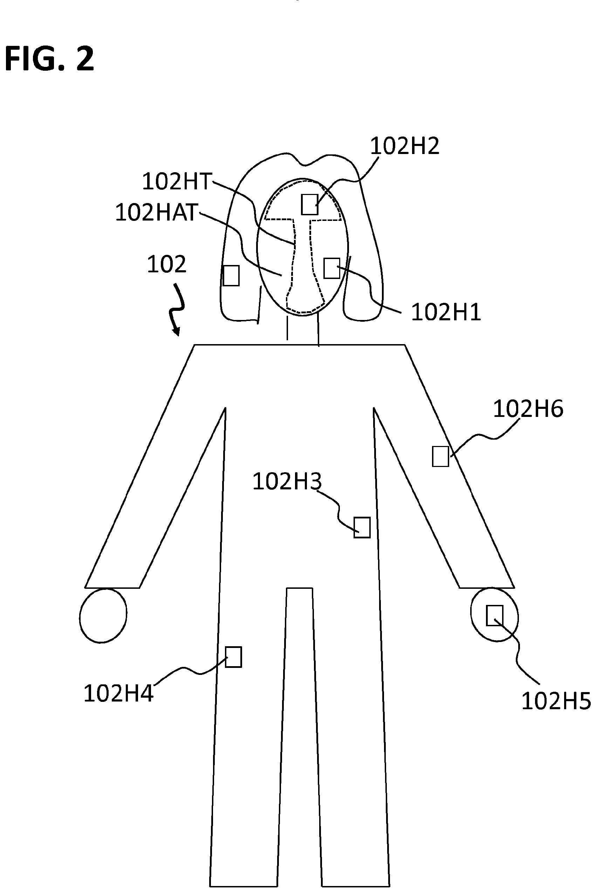

Der Hautzustand kann ermittelt werden, indem für mindestens einen Hautbereich 102H des Nutzers 102 ein Hautfettgehalt ermittelt wird und anhand dessen der Hautzustand ermittelt wird. In verschiedenen Ausführungsbeispielen kann der Hautbereich 102H ein Teil einer Gesichtshaut des Nutzers 102 sein. Alternativ oder zusätzlich kann, wie in FIG. 2 dargestellt ist, welche Hautregionen 102H zum Anwenden eines Verfahrens und einer Vorrichtung zum Ermitteln eines Hautzustands zeigt, ein anderer bzw. weiterer Hautbereich 102H, beispielsweise an einem Arm (Hautbereich 102H6), einem Bein (Hautbereich 102H4), einem Rumpf (Bereich 102H3) und/oder einer Hand (Bereich 102H5) eines Nutzers zum Ermitteln eines Hautzustands dieses Hautbereichs verwendet werden. In einem Fall, dass eine Mehrzahl von Hautbereichen 102H verwendet wird, kann mindestens einer der Mehrzahl von Hautbereichen (z.B. 102H2) in einer T-Zone 102HT des Gesichts angeordnet sein, und mindestens ein weiterer der Mehrzahl von Hautbereichen (z.B. 102H1) in einem Gesichtsbereich 102HAT außerhalb der T-Zone angeordnet sein.

Die Vorrichtung 100 weist in verschiedenen Ausführungsbeispielen eine Testfläche 104F, eine Kamera 108 und eine elektronische Schaltkreisvorrichtung 116 auf und ist eingerichtet, mittels der Testfläche 104F, der Kamera 108, der elektronischen Schaltkreisvorrichtung 116 und einer Lichtquelle 106 einen Hautfettgehalt (d.h. einen Gehalt von Hautlipiden auf/in der Haut) des Nutzers 102 zu ermitteln.

Dafür ist in verschiedenen Ausführungsbeispielen die Testfläche 104F derart gestaltet, dass sich ihre Lichtdurchlässigkeit in Abhängigkeit eines Fettgehalts von Haut, mit der sie in Kontakt gebracht wird, ändert, d.h. die Testfläche 104F kann nach dem Kontakt mit (fetthaltiger) Haut lichtdurchlässiger oder weniger lichtdurchlässig werden, und zwar in stärkerem Maße, je fetthaltiger die Haut ist.

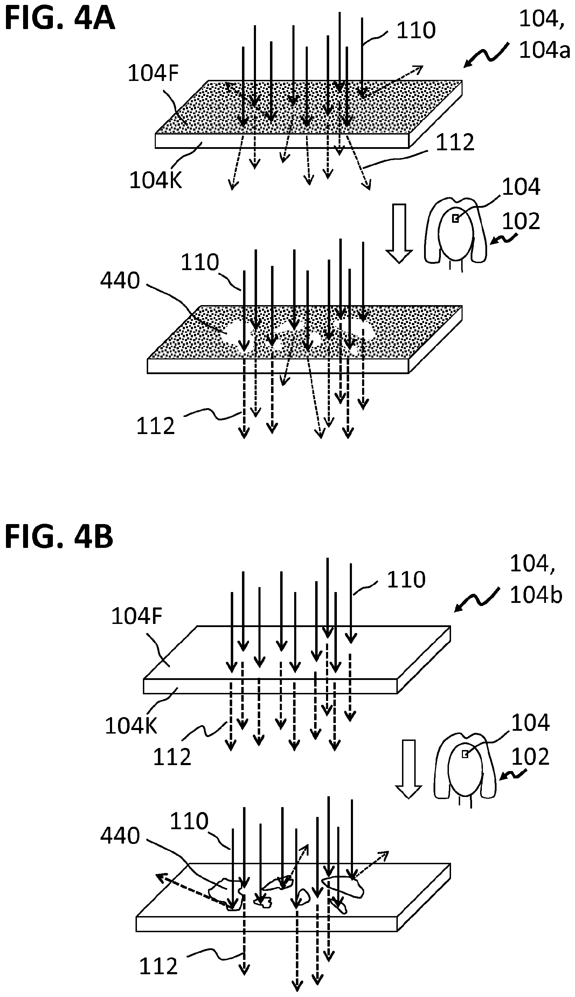

Eine Testfläche 104F (als Teil einer Testvorrichtung 104 bzw. 104a), die nach einem Kontakt mit fetthaltiger Haut (was zu einem Anhaften von Hautfett 440 auf der Testfläche 104F führt) lichtdurchlässiger wird, ist in FIG. 4A veranschaulicht. In FIG. 4A zeigt die obere Testvorrichtung 104a die Testfläche 104F vor dem Kontaktieren der Haut, und die untere Testvorrichtung 104a die Testfläche 104F nach dem Kontaktieren der Haut. Die Testfläche 104F kann beispielsweise eine raue, z.B. aufgeraute, Fläche eines ansonsten im Wesentlichen lichtdurchlässigen, z.B. transparenten, Körpers 104K sein. Der Körper 104K kann beispielsweise einen transparenten Kunststoff oder Glas aufweisen. In verschiedenen Ausführungsbeispielen kann der Körper 104 so dünn gestaltet sein (z.B. aus Kunststoff oder aus einem papierartigen Material), dass die Testfläche 104F sich im Wesentlichen durch den gesamten Körper 104 erstreckt und der gesamte Körper 104 beim Kontakt mit Hautfett lichtdurchlässiger wird, z.B. ähnlich einem Butterbrotpapier. Der Körper 104K mit der Testfläche 104F kann in verschiedenen Ausführungsbeispielen starr oder flexibel sein.

Die Fläche 104F kann beispielsweise derart rau, z.B. aufgeraut, sein, dass sie einen Teil von auf sie auftreffendem Licht 110 streut und nur einen relativ kleinen Teil des auftreffenden Lichts 110 durch die Testfläche 104F (und den Körper 104K) hindurchtreten lässt als hindurchgetretenes Licht 112 (dadurch veranschaulicht, dass hindurchgetretene Licht 112 mit weniger Pfeilen dargestellt ist, welche auch noch dünner dargestellt sind als beim auftreffenden Licht 110). In verschiedenen Ausführungsbeispielen kann es dort, wo das Hautfett 440 an der Testfläche 104F anhaftet, dem auftreffenden Licht 110 leichter möglich sein, die Testfläche 104F zu durchdringen, so dass es mehr Licht möglich ist, in Form von dem hindurchgetretenen Licht 112 durch die Testfläche 104F hindurchzutreten und die Kamera 108 zu erreichen (dadurch veranschaulicht, dass hindurchgetretene Licht 112 zwar mit weniger Pfeilen dargestellt ist als das auftreffenden Licht 110, aber mit mehr Pfeilen/dickeren Pfeilen als beim Licht 112, welches durch die unkontaktierte Testfläche 104F hindurchgetreten ist).

Eine Testfläche 104F (als Teil einer Testvorrichtung 104 bzw. 104b), die nach einem Kontakt mit fetthaltiger Haut (was zu einem Anhaften von Hautfett 440 auf der Testfläche 104F führt) weniger lichtdurchlässig (d.h. lichtundurchlässiger) wird, ist in FIG. 4B veranschaulicht. In FIG. 4B zeigt die obere Testvorrichtung 104b die Testfläche 104F vor dem Kontaktieren der Haut, und die untere Testvorrichtung 104b die Testfläche 104F nach dem Kontaktieren der Haut. Die Testfläche 104F kann beispielsweise eine glatte, saubere Fläche eines im Wesentlichen lichtdurchlässigen, z.B. transparenten, Körpers 104K sein. Der Körper 104K kann beispielsweise einen transparenten Kunststoff oder Glas aufweisen. Der Körper 104K mit der Testfläche 104F kann in verschiedenen Ausführungsbeispielen starr oder flexibel sein.

Die Fläche 104F kann beispielsweise derart gestaltet sein, dass sie einen überwiegenden Teil von auf sie auftreffendem Licht 110 hindurchtreten lässt als hindurchgetretenes Licht 112, z.B. im Wesentlichen das gesamte auf sie auftreffende Licht 110. (dadurch veranschaulicht, dass hindurchgetretene Licht 112 mit gleich vielen, gleich starken Pfeilen dargestellt ist wie das auftreffende Licht 110). In verschiedenen Ausführungsbeispielen kann es dort, wo das Hautfett 440 an der Testfläche 104F anhaftet, dem auftreffenden Licht 110 nicht möglich sein, die Testfläche 104F zu durchdringen, oder es kann dem auftreffenden Licht 110 nur möglich sein, die Testfläche 104F gestört (z.B. gestreut oder auf sonstige Weise in einer Richtung geändert) zu durchdringen, so dass es weniger Licht möglich ist, in Form von dem hindurchgetretenen Licht 112 durch die Testfläche 104F hindurchzutreten (dadurch veranschaulicht, dass hindurchgetretene Licht 112 mit weniger Pfeilen dargestellt ist als das auftreffenden Licht 110).

In verschiedenen Ausführungsbeispielen kann die Testvorrichtung 104 als wiederverwendbare Testvorrichtung 104 gestaltet sein. Zum Wiederverwenden kann die benutzte Testfläche 104F gereinigt werden, beispielsweise mittels eines Lösungsmittels für Fett, z.B. mittels Alkohols oder einer seifehaltigen Waschlösung.

In verschiedenen Ausführungsbeispielen kann die Testvorrichtung 104 als Einmal- Testvorrichtung 104 gestaltet sein, bei welcher ein Entfernen des Hautfetts unmöglich sein kann. In dem Fall kann die Vorrichtung 100 so gestaltet sein, dass an/in ihr ein Vorrat von Einmal-Testvorrichtungen 104 aufbewahrbar ist.

In verschiedenen Ausführungsbeispielen kann eine Auswahl des mindestens einen Hautbereichs 102H und das In-Kontakt-Bringen der Testfläche 102F mit der Haut wie oben beschrieben erfolgen.

Die Testfläche 104F wird oder ist nach dem In-Kontakt-Bringen mit der Haut derart angeordnet, dass die Kamera 108 der Vorrichtung eingerichtet ist, von einer Lichtquelle 106 abgestrahltes Licht 110 nach einem Hindurchtreten durch die Testfläche 104F (das durch die Testfläche 104F hindurchgetretene Licht ist in den Figuren mit 112 gekennzeichnet) zu empfangen. Anders ausgedrückt wird mittels der Kamera 108 eine Aufnahme des Lichts 112, das durch die Testfläche 104F hindurchgetreten ist, gemacht. Damit kann in verschiedenen Ausführungsbeispielen ermöglicht werden, mittels der Kamera 108 und ggf. mittels der elektronischen Schaltkreisvorrichtung 116, eine Lichtmenge zu ermitteln, welche durch die Testfläche 104F hindurchtritt, welche mit der Haut des Nutzers (d.h. einem Hautbereich 102H des Nutzers 102) in Kontakt war, und diese Lichtmenge zu vergleichen mit einer Referenzlichtmenge, welche durch eine unbenutzte, d.h. von Hautfett freie und saubere, Testfläche 104F hindurchtritt oder hindurchtreten würde. Wie oben beschrieben kann die Referenzlichtmenge in verschiedenen Ausführungsbeispielen vorab bestimmt worden und bereitgestellt sein, oder die Referenzlichtmenge kann unter Verwendung der Vorrichtung mit einer von Hautfett freien und sauberen Testfläche 104F ermittelt werden. Insbesondere in verschiedenen Ausführungsbeispielen, in welchen die Lichtquelle 106 nicht Teil der Vorrichtung 100 ist, sondern eine im Wesentlichen beliebige herkömmliche Lichtquelle 106, kann die Referenzlichtmenge mittels der Vorrichtung 100 ermittelt werden, z.B. vor oder (mit gereinigter Testfläche 104F) nach dem Ermitteln der Lichtmenge, die durch die mit Hautfett 440 versehene Testfläche 104F hindurchtritt.

Die Kamera 108 ist mit der elektronischen Schaltkreisvorrichtung 116 gekoppelt, z.B. mittels einer (kabelgebundeen oder kabellosen) Datenverbindung 118.

Die elektronische Schaltkreisvorrichtung 116 kann in verschiedenen Ausführungsbeispielen eingerichtet sein, wie oben beschrieben mittels Vergleichens der Lichtmenge, die durch die mit Hautfett 440 versehene Testfläche 104F hindurchtritt, mit der Referenzlichtmenge einen Hautfettgehalt des Nutzers 102 (bei Messung einer Mehrzahl von Hautbereichen 102H den Hautfettgehalt des jeweiligen Hautbereiches) zu ermitteln, z.B. ein sehr hoher, mäßig hoher, normaler, mäßig niedriger oder sehr niedriger Hautfettgehalt. In verschiedenen Ausführungsformen kann der Hautfettgehalt auf andere Weise beschrieben oder quantifiziert werden, z.B. als Hautfettgehaltszahl (z.B. mit einer beliebigen Einheit) oder ähnliches.

Unter Verwendung des Hautfettgehalts (bzw. der Mehrzahl von Hautfettgehalten) wird wie oben beschrieben ein Hautzustand des Nutzers 102 ermittelt.

In verschiedenen Ausführungsbeispielen kann bei nur einem ermittelten Hautfettgehalt oder bei einheitlichen ermittelten Hautfettgehalten für eine Mehrzahl unterschiedlicher Hautbereiche der Hautzustand dem ermittelten Hautfettgehalt entsprechen, z.B. kann bei einem ermittelten sehr hohen Hautfettgehalt für den einzigen, alle oder fast alle Hautbereiche der ermittelte Hautzustand sehr fettig sein, bei einem ermittelten sehr niedrigen Hautfettgehalt für den einzigen, alle oder fast alle Hautbereiche der ermittelte Hautzustand sehr trocken sein, usw.

In verschiedenen Ausführungsbeispielen kann bei uneinheitlichen ermittelten Hautfettgehalten für eine Mehrzahl unterschiedlicher Hautbereiche der Hautzustand als Mischhaut (d.h. eine Haut, die sowohl trockene als auch fettige Bereiche aufweist) ermittelt werden, wobei je nach Fettgehalt der trockenen bzw. der fettigen Bereiche unterschiedliche Arten von Mischhaut ermittelt werden können, z.B. wie oben beschrieben.

Die Vorrichtung 100 zum Ermitteln eines Hautzustands gemäß verschiedenen Ausführungsbeispielen kann eine tragbare Vorrichtung 100 sein.

Die Kamera 108 kann in verschiedenen Ausführungsbeispielen eine Kamera zum Aufnehmen von digitalen Bildern in sichtbarem Licht sein. Hierbei kann unter "sichtbarem Licht" elektromagnetische Strahlung mit einer Wellenlänge in einem Bereich von etwa 380 nm bis 780 nm zu verstehen sein. Die Kamera 108 kann geeignet sein, für das Aufnehmen der Bilder, z.B. mittels eines Detektors, Licht mindestens aus dem gesamten genannten Wellenlängenbereich zu erfassen, oder Licht aus einem oder mehreren Teilbereichen des genannten Wellenlängenbereichs.

In verschiedenen Ausführungsbeispielen, wie in FIG. 1A und FIG. 3A für die Vorrichtungen 100a und 100c dargestellt, kann es entbehrlich sein, dass die Lichtquelle 106 Teil der Vorrichtung 100 ist. Stattdessen kann, wie oben beschrieben, eine im Wesentlichen beliebige verfügbare Lichtquelle genutzt werden.

In verschiedenen Ausführungsbeispielen, wie in FIG. 1A, FIG. 1B und FIG. 3B für die Vorrichtungen 100b und 100d dargestellt, kann die Lichtquelle 106 Teil der Vorrichtung 100 sein. Beispielsweise kann in einem Fall, dass die elektronische Schaltkreisvorrichtung 116 Teil eines Smartphones, eines Tablets, eines iPads^{®} oder eines ähnlichen Geräts ist, eine üblicherweise in einem solchen Gerät vorhandene Lichtquelle als die Lichtquelle 106 genutzt werden.

In verschiedenen Ausführungsbeispielen können die Lichtquelle 106 und die Kamera 108 Teile einer integrierten tragbaren Vorrichtung sein, d.h. eine integrierte Einheit bilden. In verschiedenen Ausführungsbeispielen kann die integrierte Vorrichtung eine Größe aufweisen, die es ermöglicht, sie in einer Hand- oder Hosentasche unterzubringen. Beispielsweise kann die integrierte Vorrichtung eine Fläche von weniger als 36 cm² und eine Dicke von weniger als 2 cm aufweisen.

In verschiedenen Ausführungsbeispielen kann die Kamera 108 dafür vorgesehen sein, in einem Abstand zur Testfläche 104F angeordnet zu sein bzw. werden, wie es beispielhaft in FIG. 3A dargestellt ist. In verschiedenen Ausführungsbeispielen können beispielsweise ein Abstrahlwinkel und/oder eine Lichtintensität der Lichtquelle 106 und/oder ein Erfassungswinkel und/oder eine Empfindlichkeit der Kamera 108 so gestaltet sein, dass bei einer Anordnung der Lichtquelle 106 und der Kamera 108 mit einem Abstand zur Testfläche 104F beispielsweise bis zu einem vorgegebenen Maximalabstand ein an der Kamera 108 erfasstes Signal eine ausreichende (z.B. maximale) Intensität aufweist für ein Ermitteln des Hautfettgehalts, wie an anderer Stelle ausführlicher beschrieben. Dies kann beispielsweise dadurch erreicht werden, dass die Testfläche 104F einerseits so weit von der Kamera 108 entfernt angeordnet wird bzw. ist, dass ein möglichst großer Anteil des Bereichs der Testfläche 104F, der mit Hautfett 440 versehen ist, im Erfassungswinkel der Kamera 108 liegt, andererseits aber so nah an der Kamera 108 angeordnet wird bzw. ist, dass möglichst wenig Licht (z.B. Streulicht), welches nicht durch die Testfläche 104F hindurchgetreten ist und welches das Messergebnis verfälschen oder beeinträchtigen könnte, die Kamera 108 erreicht.

In FIG. 1A, 1B, 3A, 3B, 4A und 4B ist die Testfläche 104F rechteckig dargestellt, womit eine Anpassung an einen von der Kamera 108 erfassten Bereich erreicht werden kann. In verschiedenen Ausführungsbeispielen kann die Testfläche 104F jedoch jede beliebige Flächenform aufweisen, z.B. eine andere Polygonform, eine runde oder elliptische Form, oder ähnliches.

In verschiedenen Ausführungsbeispielen kann eine Größe der Testfläche 104F so gestaltet sein, dass sie einerseits überwiegend von der Kamera 108 erfassbar ist und andererseits für den Nutzer 102 handhabbar ist. Die Testfläche 104F kann beispielsweise eine Größe in einem Bereich von etwa 1 mm² bis etwa 6 cm² aufweisen, z.B. von etwa 1 cm² bis etwa 3 cm².

Abhängig z.B. von einer Dicke des Körpers 104K der Testvorrichtung 104 kann es nötig sein, die Testfläche 104F in einer bestimmten vorgegebenen Orientierung bezüglich der Kamera anzuordnen (entweder so, dass die Testfläche 104F zur Kamera 108 weist oder so, dass die Testfläche 104F von der Kamera 108 wegweist, z.B. in dem Fall, dass der Körper 104K vergleichsweise dick ist, z.B. mehrere Millimeter), oder die Orientierung der Testfläche 104F bezüglich der Kamera 108 kann egal sein, z.B. in dem Fall, dass der Körper 104K vergleichsweise dünn ist, z.B. eine Dicke von etwa 1 mm oder weniger aufweist.

In FIG. 3A ist der Abstand mit d gekennzeichnet und dargestellt als Abstand zwischen einem transparenten Schutzfenster vor einer Eintrittsöffnung der Kamera 108 und der Testfläche 104F.

In verschiedenen Ausführungsbeispielen, z.B. in einem Fall, in welchem die Testfläche 104F ihre Lichtdurchlässigkeit verringert, wenn sie mit dem Hautfett 440 in Kontakt gebracht wird, und das transparente Schutzfenster derart in einem Gehäuse (in FIG. 3A bzw. FIG. 3B beispielsweise das Gehäuse des Mobiltelefons) angeordnet ist, dass das Schutzfenster mit Haut in Kontakt bringbar ist (z.B. bündig mit dem Gehäuse abschließt oder leicht daraus hervorsteht, das transparente Schutzfenster die Testfläche 104F bilden (in dem Fall wäre der Abstand d Null Millimeter).

In verschiedenen Ausführungsbeispielen kann der Abstand d in einem Bereich von etwa 0 mm bis etwa 2 cm liegen, z.B. in einem Bereich von etwa 5 mm bis etwa 10 mm.

In verschiedenen Ausführungsbeispielen kann die Vorrichtung 100 zum Anordnen der Testfläche 104F in einer geeigneten Position (z.B. in der oben beschriebenen Position oder einer anderen definierten, reproduzierbaren Position) in Bezug auf die Kamera 108, z.B. derart, dass die Kamera 108 das Licht 112 von der Lichtquelle 106 empfängt, welches durch die Testfläche 104F hindurchgetreten ist, eine Halterung 330 aufweisen, wie dies z.B. in FIG. 3A und FIG. 3B dargestellt ist.

In verschiedenen Ausführungsbeispielen kann die Halterung 330 so gestaltet sein, dass sie mit der Testfläche 104F eine integrierte Einheit bildet, z.B. an der Testvorrichtung 104 befestigt ist. In dem Fall kann die Halterung 330 so gestaltet sein, dass die Testfläche 104F sich zum Kontaktieren der Haut des Nutzers 102 in einer Position befindet oder in eine solche bringbar ist, die das ermöglicht, und zum Durchstrahltwerden vom Licht 110 der Lichtquelle 106 derart, dass das durch die Testfläche 104F hindurchgetretene Licht 112 die Kamera 108 erreichen kann, sich in einer (ggf. weiteren) Position befindet oder in eine solche bringbar ist, die das ermöglicht. Dafür kann die Halterung 330 in verschiedenen Ausführungsformen mit einem beweglichen Mechanismus, z.B. einem Klapp- oder Schwenkmechanismus, versehen sein. Ferner kann in verschiedenen Ausführungsformen die Halterung 330 mit einer Schutzvorrichtung (nicht dargestellt) versehen sein, welche so angeordnet werden kann, dass sie die Testfläche 104F, z.B. bei einer Nichtbenutzung der Vorrichtung 100 schützt.

Zum Fixieren der Testfläche 104F in Bezug auf die Kamera 108 (und ggf. auch in Bezug auf die Lichtquelle 106, siehe die in FIG. 3B dargestellte beispielhafte Ausführungsform) kann die Halterung 330 eine Fixiervorrichtung aufweisen, z.B. eine Klemm-, Haft- oder Schraubvorrichtung, kann hülsenartig zum Aufschieben gestaltet sein, oder ähnliches (in FIG. 3A ist die Halterung 330 als eine Klemmvorrichtung gestaltet, in FIG. 3B als eine Haftvorrichtung).

In verschiedenen Ausführungsbeispielen kann, wie in FIG. 3B dargestellt, die Vorrichtung 100 die Lichtquelle 106 aufweisen, wobei die Kamera 108, die elektronische Schaltkreisvorrichtung 116 und die Lichtquelle 106 eine integrierte Einheit bilden können.

Insbesondere in einem Fall, in welchem ein Smartphone, ein Tablet oder ein iPad^{®} als integrierte Einheit verwendet wird, kann die integrierte Einheit herkömmlich so gestaltet sein, dass kein Licht 110 von der Lichtquelle 106 direkt die Kamera 108 erreicht. In verschiedenen Ausführungsbeispielen (z.B. in dem beschriebenen oder anderen Fällen) kann die Vorrichtung ferner ein optisches Element 332 aufweisen, wobei die Lichtquelle 116, das optische Element 332, die Testfläche 104F und die Kamera 108 derart anordenbar sind, dass für das Licht 110 der Lichtquelle 106 mittels des optischen Elements 332 die Kamera 108 nach dem Hindurchtreten durch die Testfläche 104F erreichbar ist.

Das optische Element 332 kann in verschiedenen Ausführungsbeispielen einen Spiegel (z.B. einen ebenen oder gekrümmten Spiegel) aufweisen. Ferner kann das optische Element 332 in verschiedenen Ausführungsbeispielen ein Streuelement aufweisen, um aus dem gerichteten Licht gleichmäßiges, diffuses Licht 110 zu erzeugen, eine Blende, um den beleuchteten Bereich auf der Testfläche 104F auf den Bereich zu beschränken, der von der Kamera 108 erfasst wird, und/oder weitere optische Elemente wie z.B. Linsen.

Das optische Element 332 kann in verschiedenen Ausführungsbeispielen eine integrierte Einheit mit der Halterung 330 bilden, wie in FIG. 3B dargestellt. Das optische Element 332 kann in verschiedenen Ausführungsbeispielen mit der Halterung 330 beweglich verbunden sein, so dass es beispielsweise in eine Position bringbar ist, in welcher es bei Anordnung an der integrierten Einheit von Lichtquelle 106, Kamera 108 und elektronischer Schaltkreisvorrichtung 116 das Licht 110 zur Kamera 108 richtet, und in eine zweite Position bringbar ist (z.B. mittels Klappens oder Schwenkens), in welcher die integrierte Einheit aus Halterung 330, optischem Element 332 und ggf. Testfläche 104F platzsparend aufbewahrbar ist (in welcher die integrierte Einheit aus Halterung 330, optischem Element 332 und ggf. Testfläche 104F z.B. flach ist).

Die elektronische Schaltkreisvorrichtung 116 kann in verschiedenen Ausführungsbeispielen einen Prozessor und einen Speicher aufweisen. Die elektronische Schaltkreisvorrichtung 116 kann in verschiedenen Ausführungsbeispielen mit der Lichtquelle 106 gekoppelt sein, beispielsweise derart, dass ein Datenaustausch zwischen der elektronischen Schaltkreisvorrichtung 116 und der Lichtquelle 106 ermöglicht ist, beispielsweise mittels einer Datenverbindung 118. Die Datenverbindung 118 kann beispielsweise ein Datenkabel, eine interne leitende Verbindung und/oder eine kabellose Übertragung, z.B. mittels WLAN; ZigBee, Thread oder Bluetooth^{®}, aufweisen. Mittels der Datenverbindung 118 können beispielsweise Steuerbefehle von der elektronischen Schaltkreisvorrichtung 116 an die Lichtquelle 106 übertragen werden. Die elektronische Schaltkreisvorrichtung 116 kann beispielsweise ein Smartphone, ein iPad^{®}, ein Tablet, ein Laptop oder Ähnliches sein.

Die elektronische Schaltkreisvorrichtung 116 kann in verschiedenen Ausführungsbeispielen mit der Kamera 108 gekoppelt sein, beispielsweise derart, dass ein Datenaustausch zwischen der elektronischen Schaltkreisvorrichtung 116 und der Kamera 108 ermöglicht ist, beispielsweise mittels einer Datenverbindung 118. Die Datenverbindung 118 kann beispielsweise ein Datenkabel, eine interne leitende Verbindung und/oder eine kabellose Übertragung, z.B. mittels WLAN, Thread, ZigBee oder Bluetooth^{®}, aufweisen. Mittels der Datenverbindung 118 können aufgenommene Daten, beispielsweise mindestens ein digitales Bild, von der Kamera 108 an die elektronische Schaltkreisvorrichtung 116 übertragbar sein bzw. übertragen werden.

In verschiedenen Ausführungsbeispielen (wie z.B. in FIG. 3A und FIG. 3B dargestellt) können die Lichtquelle 106, die Kamera 108 und/oder die elektronische Schaltkreisvorrichtung 116 eine tragbare integrierte Einheit bilden. Beispielsweise können die Lichtquelle 106 und die Kamera 108 eine integrierte Einheit bilden, die mit der elektronischen Schaltkreisvorrichtung 116 gekoppelt ist, oder die Lichtquelle 106 und die elektronische Schaltkreisvorrichtung 116 können eine tragbare integrierte Einheit bilden, und die Kamera 108 kann mit der Lichtquelle-Datenverarbeitungsvorrichtung gekoppelt sein, oder die Kamera 108 und die elektronische Schaltkreisvorrichtung 116 können eine tragbare integrierte Einheit bilden, und die Lichtquelle 106 kann mit der Aufnahmevorrichtung-Datenverarbeitungsvorrichtung gekoppelt sein, oder die Lichtquelle 106, die Kamera 108 und die elektronische Schaltkreisvorrichtung 116 können eine tragbare integrierte Einheit bilden.

Die elektronische Schaltkreisvorrichtung 116 kann in verschiedenen Ausführungsbeispielen eingerichtet sein, basierend auf der aufgenommenen Abbildung einen Hautfettgehalt zu ermitteln. Ferner kann in verschiedenen Ausführungsbeispielen die elektronische Schaltkreisvorrichtung eingerichtet sein, basierend auf dem mindestens einen Hautfettgehalt einen Hautzustand zu ermitteln. In verschiedenen Ausführungsbeispielen kann die elektronische Schaltkreisvorrichtung eingerichtet sein, selbst, d.h. direkt, den mindestens einen Hautzustandsparameter und/oder den Hautzustand zu ermitteln.

Für das Ermitteln des Hautfettgehalts kann in verschiedenen Ausführungsbeispielen eine Software, beispielsweise eine App, genutzt werden, welche beispielsweise auf einer tragbaren Datenverarbeitungsvorrichtung installiert sein kann. Entsprechend kann die App eine "mobile App" (Anwendungssoftware für Mobilgeräte) oder eine Web-App (Anwendungssoftware nach dem Client-Server-Modell) sein.

In verschiedenen Ausführungsbeispielen kann der Hautfettgehalt bzw. der Hautzustand zeitlich abhängig (z.B. mehrmals täglich, täglich, wöchentlich, oder mit jeder anderen zeitlichen Abhängigkeit) und/oder unter verschiedenen Umgebungsbedingungen (z.B. in einer Umgebung mit hoher oder niedriger Luftfeuchtigkeit, bei Kälte oder Hitze, uw.) ermittelt werden.

In verschiedenen Ausführungsbeispielen können Kalibrationsmessungen (auch als Referenzmessungen bezeichnet), welche beispielsweise in einem Labor durchgeführt werden können, und deren Ergebnisse in der Datenverarbeitungsvorrichtung gespeichert sein können, beispielsweise als Teil der Software/App, genutzt werden, um eine Lichtdurchlässigkeit der in-Kontakt-zur-Haut-gebrachten Testfläche einem Hautfettgehalt und/oder den mindestens einen ermittelten Hautfettgehalt einem Hautzustand zuzuordnen.

In verschiedenen Ausführungsbeispielen kann das Ermitteln des Hautfettgehalts, beispielsweise wie oben beschrieben, direkt mittels der tragbaren elektronischen Schaltkreisvorrichtung 116 ausgeführt werden, z.B. mittels der Software/App, welche auf der tragbaren elektronischen Schaltkreisvorrichtung 116 installiert sein kann.

In verschiedenen Ausführungsbeispielen kann mittels der elektronischen Schaltkreisvorrichtung, z.B. mittels der Software/App, der Hautfettgehalt bereitgestellt werden. Der Hautfettgehalt kann beispielsweise als Wert (z.B. mit willkürlichen Einheiten), als verbale Mitteilung, als graphische Darstellung, o.ä. mitgeteilt, z.B. dargestellt werden, z.B. mittels Anzeigens, z.B. an einem Bildschirm der tragbaren elektronischen Schaltkreisvorrichtung 116.

In verschiedenen Ausführungsbeispielen kann das Ermitteln des Hautfettgehalts, beispielsweise wie oben beschrieben, indirekt mittels der tragbaren elektronischen Schaltkreisvorrichtung 116 erfolgen, beispielsweise indem die elektronische Schaltkreisvorrichtung 116 das Ermitteln des Hautfettgehalts mittels einer externen Datenverarbeitungsvorrichtung 222 veranlasst. In verschiedenen Ausführungsbeispielen kann die Aufnahme und/oder das Vergleichsergebnis von der tragbaren elektronischen Schaltkreisvorrichtung 116, wie in FIG. 1B dargestellt, der externen Datenverarbeitungsvorrichtung 222, beispielsweise einem zentralen Computer oder einer Cloud, welche/r beispielsweise eine höhere Rechenleistung und/oder eine größere Speicherkapazität als die elektronische Schaltkreisvorrichtung 116 aufweisen kann, bereitgestellt werden, z.B. an diese übertragen werden. Die externe Datenverarbeitungsvorrichtung 222 kann eingerichtet sein, den Hautfettgehalt aus den bereitgestellten Daten, z.B. der Aufnahme oder des Ergebnisses des Vergleichs, zu ermitteln, beispielsweise mittels einer Software, z.B. einer App, beispielsweise wie oben beschrieben. In verschiedenen Ausführungsbeispielen kann die elektronische Schaltkreisvorrichtung 116 ferner eingerichtet sein, den Hautfettgehalt von der externen Datenverarbeitungsvorrichtung 222 zu empfangen und bereitzustellen, beispielsweise wie oben beschrieben. Zum Datenaustausch mit der externen Datenverarbeitungsvorrichtung 222 kann die elektronische Schaltkreisvorrichtung 116 in verschiedenen Ausführungsbeispielen eine Vorrichtung zur kabellosen Datenübertragungsvorrichtung aufweisen, z.B. mittels WLAN oder Bluetooth^{®}. In verschiedenen Ausführungsbeispielen kann die elektronische Schaltkreisvorrichtung 216 eingerichtet sein, die Daten mit der externen Datenverarbeitungsvorrichtung 222 mittels einer Kabelverbindung auszutauschen.

In verschiedenen Ausführungsbeispielen kann die elektronische Schaltkreisvorrichtung 116 eingerichtet sein, basierend auf dem ermittelten Hautfettgehalt einen Hautzustand zu ermitteln.

In verschiedenen Ausführungsbeispielen kann die elektronische Schaltkreisvorrichtung 116 eingerichtet sein, selbst, d.h. direkt, den mindestens einen Hautzustand zu ermitteln, beispielsweise mittels einer Software, z.B. einer App, die auf der tragbaren elektronischen Schaltkreisvorrichtung 116 installiert sein kann.

In verschiedenen Ausführungsbeispielen kann die elektronische Schaltkreisvorrichtung 116 eingerichtet sein, indirekt den mindestens einen Hautzustand zu ermitteln, beispielsweise indem die elektronische Schaltkreisvorrichtung 116 das Ermitteln des Hautzustands mittels der externen Datenverarbeitungsvorrichtung, z.B. mittels einer Software, z.B. einer App, die auf der externen Datenverarbeitungsvorrichtung 222 installiert sein kann, veranlasst, beispielsweise ähnlich wie oben für das Ermitteln des Hautfettgehalts beschrieben.

In verschiedenen Ausführungsbeispielen kann die Software/App zum Ermitteln des Hautzustandsparameters dieselbe sein wie die Software/App zum Ermitteln des Hautzustands. In verschiedenen Ausführungsbeispielen, z.B. in einem Fall, dass der mindestens eine Hautzustandsparameter mittels der externen Datenverarbeitungsvorrichtung 222 ermittelt wird, wohingegen der Hautzustand von der tragbaren elektronischen Schaltkreisvorrichtung 116 ermittelt wird, oder umgekehrt, kann/können unterschiedliche Software/Apps für das Ermitteln des Hautfettgehalts und für das Ermitteln des Hautzustands verwendet werden.

In verschiedenen Ausführungsbeispielen kann das Ermitteln des Hautzustands ein Vergleichen des mindestens einen ermittelten Hautfettgehalts mit Einträgen einer Datenbank aufweisen, welche eine Mehrzahl von Hautzuständen mit jeweils mindestens einem zugeordneten Hautfettgehalt aufweisen kann. In verschiedenen Ausführungsbeispielen kann bei einer Zuordnung von mehr als einem Hautfettgehalt zu einem Hautzustand in der Datenbank zusätzlich der Hautbereich registriert sein, für welchen der Hautfettgehalt in der Datenbank ermittelt wurde. In einem solchen Fall können beim Ermitteln des Hautzustands mittels einer Mehrzahl von Hautfettgehalten die Hautbereiche berücksichtigt werden, für welche der jeweilige Hautfettgehalt ermittelt wurde, sofern die Hautbereiche bekannt sind, z.B. vom Nutzer angegeben wurden, oder angenommen werden können, z.B. weil der Nutzer angewiesen wurde, die Testflächen mit den jeweiligen Hautbereichen in Kontakt zu bringen.

Die Datenbank kann in verschiedenen Ausführungsbeispielen in der tragbaren elektronischen Schaltkreisvorrichtung 116 und/oder in der externen Datenverarbeitungsvorrichtung 222 gespeichert sein.

Beim Ermitteln des Hautzustands kann derjenige Hautzustand als der Hautzustand des Nutzers ermittelt werden, dessen Hautfettgehalt (bzw. Hautfettgehalte) die geringsten Abweichungen zum/zu den in der Datenbank dem Hautzustand zugeordneten Hautfettgehalt(en) zeigt.

In verschiedenen Ausführungsbeispielen kann die Vorrichtung 100 eingerichtet sein, dem Nutzer 102 den ermittelten Hautzustand bereitzustellen, z.B. mittels der Software/App. Der Hautzustand kann beispielsweise als Wert (z.B. mit willkürlichen Einheiten), als verbale Mitteilung, als graphische Darstellung, o.ä. mitgeteilt, z.B. dargestellt werden, z.B. mittels Anzeigens, z.B. an einem Bildschirm der tragbaren elektronischen Schaltkreisvorrichtung 116.

Insbesondere in einem Fall, in welchem der Hautfettgehalt für eine Mehrzahl von Hautbereichen ermittelt wird, kann das Bereitstellen des Hautzustands an den Nutzer eine graphische Darstellung aufweisen, beispielsweise eine Anzeige, z.B. mittels einer Anzeigevorrichtung (z.B. eines Bildschirms/Displays).

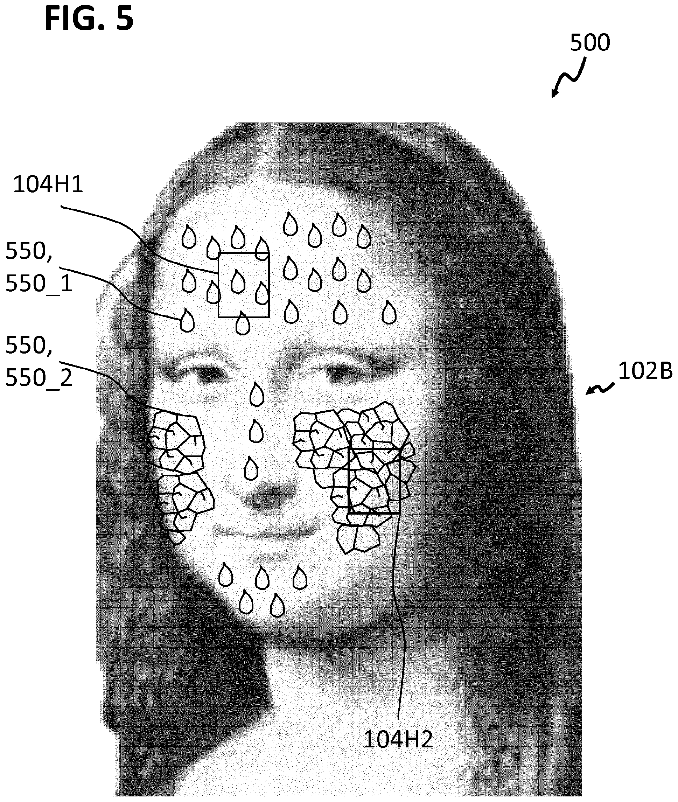

FIG. 5 zeigt beispielhaft eine Veranschaulichung einer graphischen Darstellung 500 eines Ergebnisses eines Verfahrens zum Ermitteln eines Hautzustands gemäß verschiedenen Ausführungsbeispielen.

In der graphischen Darstellung 500 kann der ermittelte Hautfettgehalt der Mehrzahl von Bereichen 104H1, 104H2 mit einer Codierung anhand des Hautfettgehalts in einer Darstellung 102B des Nutzers 102 (z.B. einer schematischen Darstellung oder auf einem Foto des Nutzers) angezeigt werden. Beispielsweise können in einer schematischen Darstellung eines Gesichts des Nutzers 102 Bereiche unterschiedlichen Hautfettgehalts mit unterschiedlichen Mustern 550 dargestellt werden, z.B. Tropfenmuster 550_1 für Bereiche mit hohem Hautfettgehalt und ein Linienmuster 550_2, welches eine aufreißende Fläche darstellt, für Bereiche mit niedrigem Hautfettgehalt, wobei Bereiche normaler Haut ungemustert verbleiben können. Alternativ kann jede beliebige andere Codierung verwendet werden, welche beispielsweise in einer Legende erläutert werden kann.

In verschiedenen Ausführungsbeispielen kann die Vorrichtung 100 ferner eingerichtet sein, basierend auf dem ermittelten Hautzustand eine kosmetischen Hautbehandlungsempfehlung zu ermitteln und dem Nutzer 102 bereitzustellen.

In verschiedenen Ausführungsbeispielen kann/können jedem der Mehrzahl von Hautzuständen mindestens ein geeignetes Pflegeprodukt und/oder mindestens ein Pflegehinweis zugeordnet sein. Die Zuordnung kann beispielsweise experimentell, z.B. bei Laborversuchen, ermittelt worden sein.

In verschiedenen Ausführungsbeispielen können zum Beurteilen einer Eignung eines Pflegeprodukts und/oder eines Pflegehinweises zum Pflegen einer Haut mit einem gegebenen Hautzustand Literaturdaten zu Grunde gelegt werden.

In verschiedenen Ausführungsbeispielen kann jedem der Hautzustände ein Qualitätswert zugeordnet sein oder werden. Ein Pflegeprodukt und/oder ein Pflegehinweis kann für einen Hautzustand als geeignet bewertet werden, wenn zu erwarten ist, z.B. aufgrund von Literaturdaten, Versuchsergebnissen oder Erfahrungswerten, dass bei einer (z.B. regelmäßigen) Anwendung des Pflegeprodukts und/oder des Pflegehinweises der Hautzustand des Nutzers 102 aufrechterhalten wird oder sich zu einem Hautzustand mit einem höheren Qualitätswert ändert.

In verschiedenen Ausführungsbeispielen, wie in FIG. 1B dargestellt, kann eine Beurteilung einer Eignung eines Pflegeproduktes, einen Hautzustand zu verbessern, bestätigt oder abgeändert werden mittels eines Aufnehmens von Erfahrungswerten weiterer Nutzer 1020 mit demselben oder einem ähnlichen Hautzustand, beispielsweise von Erfahrungswerten hinsichtlich eines Behandlungserfolgs. Die Erfahrungswerte können von den weiteren Nutzern beispielsweise der externen Datenverarbeitungsvorrichtung 222 bereitgestellt werden, beispielsweise mittels einer kabellosen Datenübertragung 226. Alternativ kann auch eine Übertragung der Daten mittels Kabel genutzt werden. Anhand der Erfahrungswerte kann die Datenbank in der externen Datenverarbeitungsvorrichtung 222 und/oder in der tragbaren elektronischen Schaltkreisvorrichtung 116 aktualisiert werden. Damit kann ermöglicht sein, dass der Nutzer 102 immer eine optimale Empfehlung erhält.

In verschiedenen Ausführungsbeispielen können beim Ermitteln der Produkt- und/oder Pflegeempfehlungen ferner Informationen hinsichtlich Alter, Geschlecht, eines generellen Gesundheitszustands, eines Hautzustands, Ernährungsgewohnheiten und weiterer Verhaltensweisen des Nutzers (z.B. tägliche Aufenthaltsdauer im Freien/in der Sonne/im Wasser, Rauchgewohnheiten usw.) verwendet werden, z.B. mittels der auf der tragbaren elektronischen Schaltkreisvorrichtung 116 und/oder auf der externen Datenverarbeitungsvorrichtung 222 installierten Software/App. Die Informationen können in verschiedenen Ausführungsbeispielen mittels der tragbaren elektronischen Schaltkreisvorrichtung 116 vom Nutzer 102 erfragt werden, welcher sie in die elektronische Schaltkreisvorrichtung 116 eingeben kann, beispielsweise mittels einer Tastatur, als Sprachnachricht, als Auswahl aus einem von der tragbaren elektronischen Schaltkreisvorrichtung 116 dargestellten Menü, oder ähnliches.

Beispielsweise können, wenn der Nutzer 102 als zusätzliche Information zur Verfügung stellt, dass er viel Zeit im Wasser oder im Freien verbringt, bei der Produkt- und/oder Pflegehinweisempfehlung basierend auf dem Hautzustand des Nutzers 102 beispielsweise diejenigen zugeordneten Pflegeprodukte dem Nutzer 102 empfohlen werden, die nicht nur für seinen Hautzustand geeignet sind, sondern außerdem z.B. wasserfest und/oder mit einem UV-Filter versehen sind.

In verschiedenen Ausführungsbeispielen kann die Produktempfehlung und/oder der Pflegehinweis dem Nutzer 102 bereitgestellt werden, beispielsweise mittels der elektronischen Schaltkreisvorrichtung 116, z.B. mittels des Bildschirms der elektronischen Schaltkreisvorrichtung 116. Beispielsweise kann die graphische Darstellung 500 aus FIG. 5 durch (z.B. Text-)Mitteilungen ergänzt werden (nicht dargestellt), z.B. die ermittelten Produktempfehlungen und/oder Pflegehinweise, wie beispielsweise "Trage Creme A im Bereich mit dem Tropfenmuster auf", "Verwende Reinigungslotion B im gesamten Gesichtsbereich", oder ähnliches.

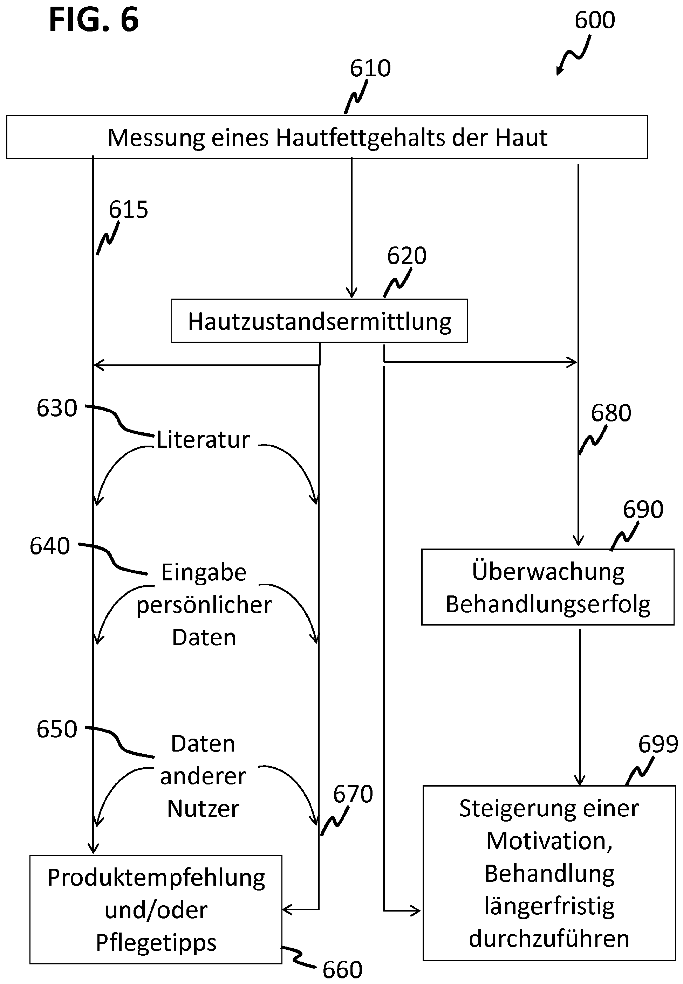

FIG. 6 zeigt ein Diagramm 600 zum Veranschaulichen eines Verfahrens zum Ermitteln eines Kosmetikprodukts zur Hautbehandlung. FIG. 6 kann im Wesentlichen diejenigen Prozesse veranschaulichen, die an anderer Stelle im Zusammenhang mit dem Verfahren zum Ermitteln eines Kosmetikprodukts zur Hautbehandlung beschrieben sind.

Das Verfahren kann aufweisen ein Ermitteln eines Hautfettgehalts der Haut (bei 610), beispielsweise wie oben beschrieben.

Das Verfahren kann ferner ein Ermitteln eines Hautzustands (auch als Hauttyp bezeichnet) aufweisen (bei 620), beispielsweise wie oben beschrieben.

Basierend auf dem ermittelten Hautzustand und/oder auf dem ermittelten Hautfettgehalt kann eine Produkt- oder Pflegetippempfehlung ermittelt werden (bei 660). Dabei kann (als Pfad 670 gekennzeichnet) nur der anhand des Hautfettgehalts ermittelte Hautzustand verwendet werden.

Andererseits (als Pfad 615 gekennzeichnet) können zusätzlich zum Hautfettgehalt auch Literaturdaten (mit 630 gekennzeichnet), die persönlichen Daten (z.B. wie oben beschrieben, mit 640 gekennzeichnet), und oder Daten anderer Nutzer, z.B. deren Erfahrungswerte (als 650 gekennzeichnet) hinzugezogen werden.

Ferner kann, wie bei Pfad 680 dargestellt, mittels eines Ermittelns des Hautfettgehalts (bei 610) ein Behandlungserfolg überwacht werden (bei 690). Dies kann beispielsweise besonders während und nach einer kosmetischen Behandlung aufgrund der Produktempfehlung (bei 660) sinnvoll sein. Ein mittels des Verfahrens zum Ermitteln des Hautzustands anhand objektiver Werte belegter Behandlungserfolg kann eine Motivation beim Nutzer steigern (bei 699).

Wie oben beschrieben, können anhand des ermittelten Hautfettgehalts und/oder anhand des ermittelten Hautzustands Produktempfehlungen für individuell für den Nutzer 102 passende Pflegeprodukte und/oder individuelle Pflegehinweise abgeleitet werden. Die Produktempfehlungen und/oder Pflegehinweise können beispielsweise mittels einer Software, z.B. einer App, bereitgestellt werden.

Die Software/App, welche den Hautzustandsparameter und/oder den Hautzustand ermittelt, kann dieselbe sein, die die Produktempfehlung und/oder die Pflegehinweise ermittelt. In verschiedenen Ausführungsbeispielen können unterschiedliche Softwareprogramme/Apps verwendet werden für einen Teil der verschiedenen Vorgänge oder alle verschiedenen Vorgänge (Ermitteln des Hautfettgehalts, Ermitteln des Hautzustands, Ermitteln einer Produktempfehlung, Ermitteln eines Pflegehinweises).

Ferner kann ein Behandlungserfolg bei einer kosmetischen Behandlung, welche ein positives Beeinflussen der ermittelten Hautzustandsparameter bzw. des ermittelten Hautzustands zum Ziel haben kann, überwacht werden. In verschiedenen Ausführungsbeispielen kann die Software/App eine Kontrolle und/oder Nachverfolgung der Ergebnisse mittels einer Darstellung (z.B. einer graphischen Darstellung) der Messergebnisse im Verlauf der Zeit ermöglichen.

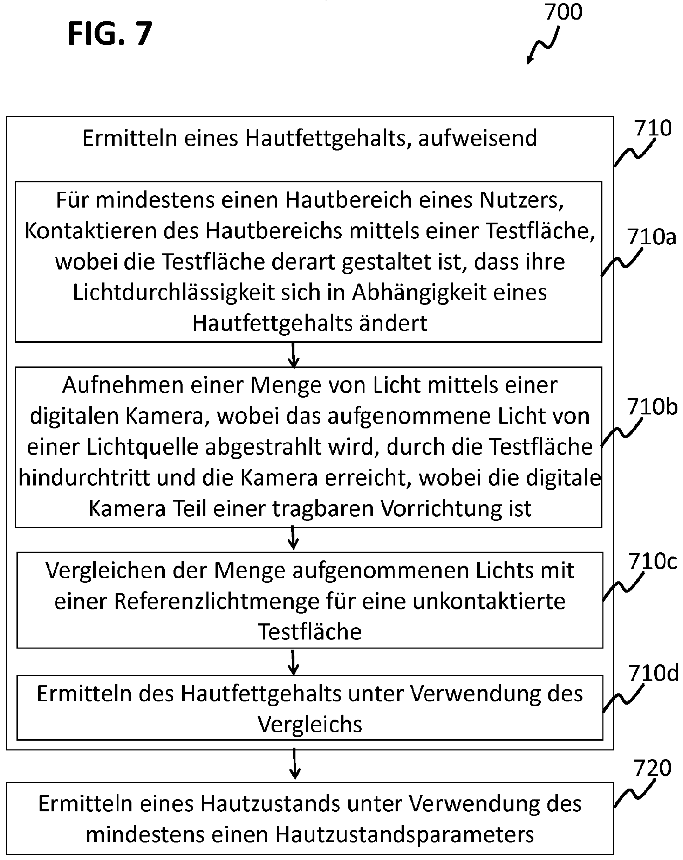

FIG. 7 zeigt ein Flussdiagramm 700 eines Verfahrens zum Ermitteln eines Hautzustands.

Das Verfahren kann aufweisen ein Ermitteln eines Hautfettgehalts (bei 710), aufweisend: für mindestens einen Hautbereich eines Nutzers, Kontaktieren des Hautbereichs mittels einer Testfläche, wobei die Testfläche derart gestaltet ist, dass ihre Lichtdurchlässigkeit sich in Abhängigkeit eines Hautfettgehalts ändert (bei 710a), Aufnehmen einer Menge von Licht mittels einer digitalen Kamera, wobei das aufgenommene Licht von einer Lichtquelle abgestrahlt wird, durch die Testfläche hindurchtritt und die Kamera erreicht, wobei die digitale Kamera Teil einer tragbaren Vorrichtung ist (bei 710b), Vergleichen der Menge des aufgenommenen Lichts mit einer Referenzlichtmenge für eine unkontaktierte Testfläche (bei 710c), und Ermitteln des Hautfettgehalts unter Verwendung des Vergleichs (bei 710d), und Ermitteln eines Hautzustands unter Verwendung des mindestens einen Hautzustandsparameters (bei 720).

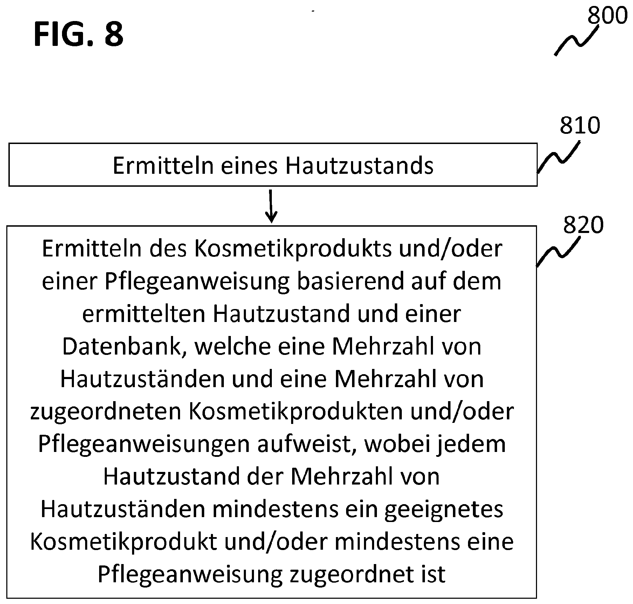

FIG. 8 zeigt ein Flussdiagramm 800 eines Verfahrens zum Ermitteln eines Kosmetikprodukts.

Das Verfahren kann aufweisen: ein Ermitteln eines Hautzustands gemäß verschiedenen Ausführungsbeispielen (bei 810) und ein Ermitteln des Kosmetikprodukts basierend auf dem ermittelten Hautzustand und einer Datenbank, welche eine Mehrzahl von Hautzuständen und eine Mehrzahl von zugeordneten Kosmetikprodukten aufweist, wobei jedem Hautzustand der Mehrzahl von Hautzuständen mindestens ein geeignetes Kosmetikprodukt zugeordnet sein kann (bei 820).

Manche der Ausführungsbeispiele sind im Zusammenhang mit Vorrichtungen beschrieben, und manche der Ausführungsbeispiele sind im Zusammenhang mit Verfahren beschrieben. Weitere vorteilhafte Ausgestaltungen des Verfahrens ergeben sich aus der Beschreibung der Vorrichtung und umgekehrt.

## Patentansprüche

1. Verfahren zum Ermitteln eines individuell für einen Nutzer (102) abgeleiteten Pflegeprodukts anhand eines ermittelten Hautzustands, aufweisend:
Ermitteln eines Hautfettgehalts, aufweisend:
für mindestens einen Hautbereich (102H) eines Nutzers (102), Kontaktieren des Hautbereichs (102H) mittels einer Testfläche (104F), wobei die Testfläche (104F) derart gestaltet ist, dass ihre Lichtdurchlässigkeit sich in Abhängigkeit eines Hautfettgehalts ändert;
Aufnehmen einer Menge von Licht mittels einer digitalen Kamera (108), wobei das aufgenommene Licht von einer Lichtquelle (106) abgestrahlt wird, durch die Testfläche (104F) hindurchtritt und die Kamera (108) erreicht, wobei die digitale Kamera (108) Teil einer tragbaren Vorrichtung ist;
Vergleichen der Menge aufgenommenen Lichts mit einer Referenzlichtmenge für eine unkontaktierte Testfläche (104F);
Ermitteln des Hautfettgehalts unter Verwendung des Vergleichs;
Ermitteln des Hautzustands basierend auf dem ermittelten Hautfettgehalt; und
Ermitteln des Pflegeprodukts mittels eines Produktkonfigurators, wobei der Produktkonfigurator konfiguriert ist, eine Auswahl für den ermittelten Hautfettgehalt und/oder Hautzustand geeigneter Inhaltsstoffe auszugeben und die Auswahl chemisch und/oder physikalisch inkompatibler Inhaltsstoffe oder die Auswahl für den ermittelten Hautfettgehalt und/oder Hautzustand ungeeigneter Inhaltsstoffe zu vermeiden.

2. Verfahren gemäß Anspruch 1,
wobei das Ändern der Lichtdurchlässigkeit ein Erhöhen der Lichtdurchlässigkeit mit Erhöhung des Hautfettgehalts aufweist.

3. Verfahren gemäß Anspruch 1,
wobei das Ändern der Lichtdurchlässigkeit ein Verringern der Lichtdurchlässigkeit mit Erhöhung des Hautfettgehalts aufweist.

4. Verfahren gemäß einem der Ansprüche 1 bis 3,
wobei der mindestens eine Hautbereich (102H) eine Mehrzahl von Hautbereichen des Nutzers (102) aufweist.

5. Verfahren gemäß Anspruch 4,
wobei Ermitteln des Hautfettgehalts für die Mehrzahl von Hautbereichen (102H) aufweist:
Kontaktieren eines ersten Hautbereichs (102H) mittels der Testfläche (104F);
Aufnehmen einer ersten Menge von Licht mittels der digitalen Kamera (108), wobei das aufgenommene Licht von der Lichtquelle (106) abgestrahlt wird, durch die Testfläche (104F) hindurchtritt und die Kamera (108) erreicht;
Reinigen der Testfläche (104F);
Kontaktieren eines zweiten Hautbereichs mittels der gereinigten Testfläche (104F);
Aufnehmen einer zweiten Menge von Licht mittels der digitalen Kamera (108), wobei das aufgenommene Licht von der Lichtquelle (106) abgestrahlt wird, durch die Testfläche (104F) hindurchtritt und die Kamera (108) erreicht.

6. Verfahren gemäß einem der Ansprüche 1 bis 5, ferner aufweisend:
vor dem Kontaktieren des Hautbereichs (102H) mittels der Testfläche (104F), Aufnehmen der Referenzlichtmenge mittels der digitalen Kamera (108), wobei das aufgenommene Licht von der Lichtquelle (106) abgestrahlt wird, durch die unkontaktierte Testfläche (104F) hindurchtritt und die Kamera (108) erreicht.

7. Verfahren gemäß einem der Ansprüche 1 bis 6,
wobei das Aufnehmen der Menge von Licht, das Vergleichen der Menge des aufgenommenen Lichts mit der Referenzlichtmenge, das Ermitteln des Hautfettgehalts und/ oder das Ermitteln des Hautzustands basierend auf dem ermittelten Hautfettgehalt mittels einer App erfolgt.

8. Verfahren gemäß einem der Ansprüche 1 bis 7,
wobei das Ermitteln des Hautfettgehalts und/oder das Ermitteln des Hautzustands ein Übertragen der Aufnahme, der ermittelten Menge aufgenommenen Lichts, eines Ergebnisses des Vergleichs der Menge aufgenommenen Lichts mit der Referenzlichtmenge und/oder des ermittelten Hautfettgehalts an eine externe Datenverarbeitungsvorrichtung (222) und ein Empfangen des Hautfettgehalts und/oder des Hautzustands aufweist.

9. Verfahren gemäß Anspruch 1,
wobei die Testfläche (104F) reinigbar und nach einem Reinigen als Testfläche (104F) wiederverwendbar ist.

10. Vorrichtung (100a, 100b, 100c,100d) zum Ermitteln eines individuell für einen Nutzer (102) abgeleiteten Pflegeprodukt anhand eines ermittelten eines Hautzustands, aufweisend:
eine Testfläche (104F), welche derart gestaltet ist, dass sie geeignet ist, mit einem Hautbereich (102H) eines Nutzers (102) in Kontakt gebracht zu werden, und dass ihre Lichtdurchlässigkeit sich in Abhängigkeit eines Hautfettgehalts ändert;
eine digitale Kamera (108), welche Teil einer tragbaren Vorrichtung und geeignet ist, Licht aufzunehmen, welches von einer Lichtquelle (106) abgestrahlt wird, wobei die Lichtquelle (106), die Testfläche (104F) und die Kamera (108) derart anordenbar sind, dass das aufgenommene Licht die Kamera (108) erreicht, nachdem es durch die Testfläche (104F) hindurchgetreten ist; eine elektronische Schaltkreisvorrichtung (116), welche eingerichtet ist, die Menge aufgenommenen Lichts mit einer Referenzlichtmenge für eine unkontaktierte Testfläche (104F) zu vergleichen, einen Hautfettgehalt unter Verwendung des Vergleichs zu ermitteln und den Hautzustand basierend auf dem ermittelten Hautfettgehalt zu ermitteln,
ein Produktkonfigurator zum Ermitteln des Pflegeprodukts, wobei der Produktkonfigurator konfiguriert ist, eine Auswahl für den ermittelten Hautfettgehalt und/oder Hautzustand geeigneter Inhaltsstoffe auszugeben und die Auswahl chemisch und/oder physikalisch inkompatibler Inhaltsstoffe oder die Auswahl für den ermittelten Hautfettgehalt und/oder Hautzustand ungeeigneter Inhaltsstoffe zu vermeiden.

11. Vorrichtung (100a, 100b, 100c,100d) gemäß Anspruch 10,
wobei die tragbare Vorrichtung ein Smartphone, ein Tablet oder ein iPad aufweist.

12. Vorrichtung (100a, 100b, 100c,100d) gemäß Anspruch 10
wobei die Testfläche (104F) reinigbar und nach einem Reinigen als Testfläche (104F) wiederverwendbar ist.

## Claims

1. A method for determining a care product individually derived for a user (102), based on a determined condition of the skin, comprising: determining a skin lipid content, comprising:
for at least one skin region (102H) of a user (102), contacting the skin region (102H) using a test surface (104F), wherein the test surface (104F) is designed such that its transparency changes depending on a skin lipid content;
recording an amount of light using a digital camera (108), wherein the recorded light is emitted by a light source (106), passes through the test surface (104F), and reaches the camera (108), wherein the digital camera (108) is part of a portable device;
comparing the amount of light recorded with a reference light amount for a non-contacted test surface (104F);
determining skin lipid content using the comparison;
determining the condition of the skin based on the determined skin lipid content; and
determining the care product by means of a product configurator, wherein the product configurator is configured to output a selection of ingredients suitable for the determined skin lipid content and/or condition of the skin, and to avoid the selection of chemically and/or physically incompatible ingredients, or the selection of ingredients unsuitable for the determined skin lipid content and/or condition of the skin.

2. The method according to claim 1,
wherein the change in transparency comprises an increase in transparency with an increase in the skin lipid content.

3. The method according to claim 1,
wherein the change in transparency comprises a decrease in transparency with an increase in the skin lipid content.

4. The method according to one of claims 1 to 3,
wherein the at least one skin region (102H) comprises a plurality of skin regions of the user (102).

5. The method according to claim 4,
wherein determining the skin lipid content for the plurality of skin regions (102H) comprises: contacting a first skin region (102H) using the test surface (104F);
recording a first amount of light using the digital camera (108), wherein the recorded light is emitted by the light source (106), passes through the test surface (104F) and reaches the camera (108);
cleaning the test surface (104F);
contacting a second skin region using the cleaned test surface (104F); recording a second amount of light using the digital camera (108), wherein the recorded light is emitted by the light source (106), passes through the test surface (104F), and reaches the camera (108).

6. The method according to one of claims 1 to 5, further comprising:
recording the reference light amount using the digital camera (108) before contacting the skin region (102H) using the test surface (104F), wherein the recorded light is emitted by the light source (106), passes through the non-contacted test surface (104F) and reaches the camera (108).

7. The method according to one of claims 1 to 6,
wherein recording the amount of light, comparing the amount of recorded light with the reference light amount, determining the skin lipid content and/or determining the condition of the skin based on the determined skin lipid content is carried out by means of an app.

8. The method according to one of claims 1 to 7,
wherein determining the skin lipid content and/or determining the condition of the skin comprises transmitting the recording, the determined amount of recorded light, a result of the comparison of the amount of recorded light with the reference light amount, and/or the determined skin lipid content to an external data processing device (222), and receiving the skin lipid content and/or the condition of the skin.

9. The method according to claim 1,
wherein the test surface (104F) can be cleaned and is reusable as the test surface (104F) after cleaning.

10. A device (100a, 100b, 100c, 100d) for determining a care product individually derived for a user (102) based on a determined condition of the skin, comprising:
a test surface (104F) which is designed such that the test surface is suitable to be brought into contact with a skin region (102H) of a user (102), and such that its transparency changes depending on a skin lipid content;
a digital camera (108) which is part of a portable device and is suitable for recording light emitted by a light source (106), wherein the light source (106), the test surface (104F), and the camera (108) can be arranged such that the recorded light reaches the camera (108) after passing through the test surface (104F); an electronic circuit device (116) which is configured to compare the amount of recorded light with a reference light amount for a non-contacted test surface (104F), to determine a skin lipid content using the comparison, and to determine the condition of the skin based on the determined skin lipid content,
a product configurator for determining the care product, wherein the product configurator is configured to output a selection of ingredients suitable for the determined skin lipid content and/or condition of the skin, and to avoid the selection of chemically and/or physically incompatible ingredients, or the selection of ingredients unsuitable for the determined skin lipid content and/or condition of the skin.

11. The device (100a, 100b, 100c, 100d) according to claim 10,
wherein the portable device comprises a smartphone, a tablet or an iPad.

12. The device (100a, 100b, 100c, 100d) according to claim 10,
wherein the test surface (104F) can be cleaned and is reusable as the test surface (104F) after cleaning.

## Revendications

1. Procédé permettant de déterminer un produit de soin déduit individuellement pour un utilisateur (102) à l'aide d'un état de la peau déterminé, présentant : la détermination d'une teneur en graisse de la peau, présentant :
pour au moins une zone de peau (102H) d'un utilisateur (102), la mise en contact de la zone de peau (102H) avec une surface de test (104F), dans lequel la surface de test (104F) est conçue de telle sorte que sa translucidité varie en fonction d'une teneur en graisse de la peau ;
la détection d'une quantité de lumière au moyen d'une caméra (108) numérique, dans lequel la lumière détectée est émise par une source de lumière (106), traverse la surface de test (104F) et atteint la caméra (108), dans lequel la caméra (108) numérique fait partie d'un dispositif portatif ;
la comparaison de la quantité de lumière détectée avec une quantité de lumière de référence pour une surface de test (104F) non mise en contact ;
la détermination du taux de graisse de la peau à l'aide de la comparaison ;
la détermination de l'état de la peau sur la base de la teneur en graisse de la peau déterminée ; et
la détermination du produit de soin au moyen d'un dispositif de configuration de produit, dans lequel le dispositif de configuration de produit est configuré pour émettre une sélection d'ingrédients appropriés pour le taux de graisse de la peau et/ou pour l'état de la peau déterminés et pour éviter la sélection d'ingrédients chimiquement et/ou physiquement incompatibles ou la sélection d'ingrédients inappropriés pour le taux de graisse de la peau et/ou l'état de la peau déterminés.

2. Procédé selon la revendication 1,
dans lequel la variation de la translucidité présente une augmentation de la translucidité avec une augmentation de la teneur en graisse de la peau.

3. Procédé selon la revendication 1,
dans lequel la variation de la translucidité présente une diminution de la translucidité avec une augmentation de la teneur en graisse de la peau.

4. Procédé selon l'une des revendications 1 à 3,
dans lequel l'au moins une zone de peau (102H) présente une pluralité de zones de peau de l'utilisateur (102).

5. Procédé selon la revendication 4,
dans lequel la détermination de la teneur en graisse de la peau présente pour la pluralité de zones de peau (102H) : la mise en contact d'une première zone de peau (102H) avec la surface de test (104F) ;
la détection d'une première quantité de lumière au moyen de la caméra (108) numérique, dans lequel la lumière détectée est émise par la source de lumière (106), traverse la surface de test (104F) et atteint la caméra (108) ;
le nettoyage de la surface de test (104F) ;
la mise en contact d'une seconde zone de peau avec la surface de test (104F) nettoyée ; la détection d'une seconde quantité de lumière au moyen de la caméra (108) numérique, dans lequel la lumière détectée est émise par la source de lumière (106), traverse la surface de test (104F) et atteint la caméra (108).

6. Procédé selon l'une des revendications 1 à 5, présentant en outre :
avant la mise en contact de la zone de peau (102H) avec la surface de test (104F), la détection de la quantité de lumière de référence au moyen de la caméra (108) numérique, dans lequel la lumière détectée est émise par la source de lumière (106), traverse la surface de test (104F) non mise en contact et atteint la caméra (108).

7. Procédé selon l'une des revendications 1 à 6,
dans lequel la détection de la quantité de lumière, la comparaison de la quantité de lumière détectée avec la quantité de lumière de référence, la détermination de la teneur en graisse de la peau et/ou la détermination de l'état de la peau sur la base de la teneur en graisse de la peau déterminée sont effectuées au moyen d'une application.

8. Procédé selon l'une des revendications 1 à 7,
dans lequel la détermination de la teneur en graisse de la peau et/ou la détermination de l'état de la peau présentent une transmission de la détection, de la quantité déterminée de lumière détectée, d'un résultat de la comparaison de la quantité de lumière détectée avec la quantité de lumière de référence et/ou de la teneur en graisse de la peau déterminée à un dispositif de traitement de données externe (222) et une réception de la teneur en graisse de la peau et/ou de l'état de la peau.

9. Procédé selon la revendication 1,
dans lequel la surface de test (104F) peut être nettoyée et peut être réutilisée comme surface de test (104F) après un nettoyage.

10. Dispositif (100a, 100b, 100c, 100d) permettant de déterminer un produit de soin déduit individuellement pour un utilisateur (102) à l'aide d'un état de la peau déterminé, présentant :
une surface de test (104F) conçue de telle sorte qu'elle est appropriée pour être mise en contact avec une zone de peau (102H) d'un utilisateur (102) et que sa translucidité varie en fonction d'une teneur en graisse de la peau ;
une caméra (108) numérique faisant partie d'un dispositif portatif et étant appropriée pour détecter une lumière émise par une source de lumière (106), dans lequel la source de lumière (106), la surface de test (104F) et la caméra (108) peuvent être disposées de telle sorte que la lumière détectée atteint la caméra (108) après avoir traversé la surface de test (104F) ; un dispositif de circuit de commutation (116) électronique configuré pour comparer la quantité de lumière détectée avec une quantité de lumière de référence pour une surface de test (104F) non mise en contact, pour déterminer une teneur en graisse de la peau à l'aide de la comparaison, et pour déterminer l'état de la peau sur la base de la teneur en graisse de la peau déterminée,
un dispositif de configuration de produit permettant de déterminer un produit de soin, dans lequel le dispositif de configuration de produit est configuré pour émettre une sélection d'ingrédients appropriés pour le taux de graisse de la peau et/ou pour l'état de la peau déterminés et pour éviter la sélection d'ingrédients chimiquement et/ou physiquement incompatibles ou la sélection d'ingrédients inappropriés pour le taux de graisse de la peau et/ou l'état de la peau déterminés.

11. Dispositif (100a, 100b, 100c, 100d) selon la revendication 10,
dans lequel le dispositif portatif présente un mobile multifonction, une tablette ou un iPad.

12. Dispositif (100a, 100b, 100c, 100d) selon la revendication 10,
dans lequel la surface de test (104F) peut être nettoyée et peut être réutilisée comme surface de test (104F) après un nettoyage.
